(19) Europäisches Patentamt
European Patent Office
Office européen des brevets

(11) Veröffentlichungsnummer: **0 190 611**
**A2**

(12) **EUROPÄISCHE PATENTANMELDUNG**

(21) Anmeldenummer: **86100837.3**

(22) Anmeldetag: **22.01.86**

(51) Int. Cl.⁴: **C 07 C 127/22**
C 07 C 157/12, A 01 N 47/34

(30) Priorität: **08.02.85 DE 3504749**
**21.03.85 DE 3510556**
**15.08.85 DE 3529343**

(43) Veröffentlichungstag der Anmeldung:
**13.08.86 Patentblatt 86/33**

(84) Benannte Vertragsstaaten:
**AT BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Wegner, Peter, Dr.**
**Paderborner Strasse 7**
**D-1000 Berlin 15(DE)**

(72) Erfinder: **Krüger, Hans-Rudolf, Dr.**
**Kulmbacher Strasse 15**
**D-1000 Berlin 30(DE)**

(72) Erfinder: **Franke, Heinrich, Dr.**
**Fabeckstrasse 38**
**D-1000 Berlin 33(DE)**

(72) Erfinder: **Joppien, Hartmut, Dr.**
**Juttastrasse 18**
**D-1000 Berlin 37(DE)**

(54) **Acylharnstoffe, insektizide Mittel enthaltend diese Verbindungen sowie Verfahren zu ihrer Herstellung.**

(57) Die Erfindung betrifft neue Acylharnstoffe der allgemeinen Formel

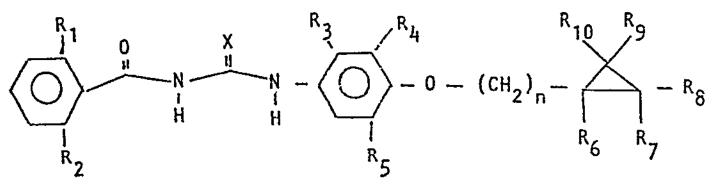

I

in der
$R_1$ Halogen, $C_1$-$C_6$alkyl oder $C_1$-$C_6$-Halogenalkyl,
$R_2$ Wasserstoff oder Halogen,
$R_3$, $R_4$ und $R_5$ jeweils Wasserstoff, Halogen oder $C_1$-$C_6$-Alkyl,
$R_6$, $R_7$ und $R_8$ jeweils Wasserstoff, $C_1$-$C_6$-Alkyl, Aryl oder Halogen,
$R_9$, $R_{10}$ gleich oder verschieden sind und Wasserstoff oder Fluor,
X Sauerstoff oder Schwefel und
n 0, 1, 2 oder 3 bedeuten,
insektizide Mittel enthaltend diese Verbindungen sowie Verfahren zu ihrer Herstellung.

EP 0 190 611 A2

Croydon Printing Company Ltd.

Die Erfindung betrifft neue Acylharnstoffe, insektizide Mittel enthaltend diese Verbindungen sowie Verfahren zu ihrer Herstellung.

Es ist bereits bekannt, daß bestimmte Acylharnstoffe insektizide Eigenschaften besitzen (DE-OS 21 23 236; DE-OS 26 01 780; DE-OS 29 28 410).

Aufgabe der vorliegenden Erfindung ist die Zurverfügungstellung von Verbindungen, die Insekten wesentlich besser bekämpfen als die für diesen Zweck bekannten Verbindungen.

Es wurde nun gefunden, daß Verbindungen der allgemeinen Formel

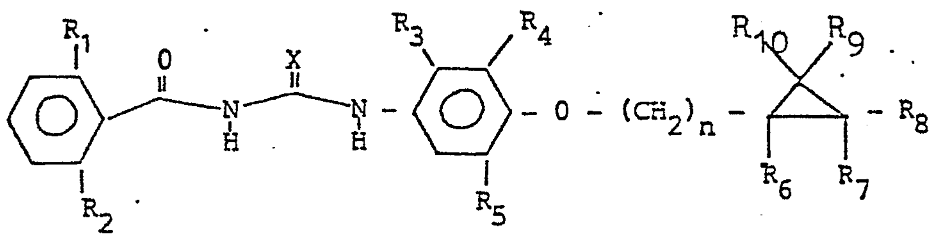

I

in der

$R_1$ Halogen, $C_1$-$C_6$Alkyl oder $C_1$-$C_6$-Halogenalkyl,
$R_2$ Wasserstoff oder Halogen,
$R_3$, $R_4$ und $R_5$ jeweils Wasserstoff, Halogen oder $C_1$-$C_6$-Alkyl,
$R_6$, $R_7$ und $R_8$ jeweils Wasserstoff, $C_1$-$C_6$-Alkyl, Aryl oder Halogen,
$R_9$ und $R_{10}$ gleich oder verschieden sind und Wasserstoff oder Fluor,
X Sauerstoff oder Schwefel und
n 0, 1, 2 oder 3

bedeuten, eine wesentlich bessere insektizide Wirkung als bekannte, wirkungsähnliche Verbindungen analoger Konstitution besitzen.

Überraschenderweise lassen sich mit den erfindungsgemäßen Verbindungen bedeutende Insektenschädlinge, insbesondere aus den Ordnungen der Dipteren, Coleopteren, Lepidopteren und Hymenopteren selektiv bekämpfen.

- 2 -

Postanschrift: Schering Aktiengesellschaft, Postfach 65 03 11, D-1000 Berlin 65 · Fur Besucher. Berlin-Wedding, Mullerstraße 170-178 Telegramme. Scheringchemie Berlin

Vorstand: Dr. Herbert Asmis Dr. Christian Bruhn, Dr. Heinz Hannse, Horst Kramp, Dr. Klaus Pohle, Dr. Horst Witzel Vorsitzender des Aufsichtsrats: Hans-Jürgen Hamann Sitz der Gesellschaft Berlin und Bergkamen · Handelsregister: AG Charlottenburg 93 HRB 283 und AG Kamen HRB 0061 Berliner Commerzbank AG Berlin Konto-Nr 108700600 Bankleitzahl 100 400 00 · Berliner Handels- und Frankfurter Bank, Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr. 1175-101 Bankleitzahl 100 100 10

Die Bezeichnung $C_1$-$C_6$-Alkyl umfaßt zum Beispiel die Radikale Methyl, Äthyl, Propyl, Isopropyl, n-Butyl, Isobutyl, n-Pentyl, n-Hexyl und andere.

Aryl soll zum Beispiel Phenyl oder Naphthyl gegebenenfalls substituiert zum Beispiel durch Halogen, wie Chlor, oder Alkyl, wie Methyl, bedeuten. Halogen kann Fluor, Chlor, Brom oder Jod darstellen.

Als Verbindungen mit besonderer insektizider Wirkung sind solche zu nennen, bei denen in der allgemeinen Formel I

$R_1$ Chlor oder Fluor,
$R_2$ Wasserstoff, Chlor oder Fluor,
$R_3$ Wasserstoff, Chlor oder Methyl,
$R_4$ Wasserstoff, Chlor oder Methyl,
$R_5$ Wasserstoff, Chlor oder Methyl,
$R_6$ Wasserstoff, Chlor oder Methyl,
$R_7$ Wasserstoff oder Methyl,
$R_8$ Wasserstoff oder Methyl,
$R_9$ und $R_{10}$ Fluor,
X Sauerstoff und
n 0, 1, 2 und 3 bedeuten.

Die Anwendung der erfindungsgemäßen Verbindungen kann in Konzentrationen von 0;0005 bis 5,0 %, vorzugsweise von 0,001 bis 0,1 % erfolgen.

- 3

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Heinz Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West Konto-Nr. 1175-101 Bankleitzahl 100 100 10

Die erfindungsgemäßen Verbindungen können entweder allein, in Mischung miteinander oder mit anderen insektiziden Wirkstoffen angewendet werden. Gegebenenfalls können andere Pflanzenschutz- oder Schädlingsbekämpfungsmittel wie zum Beispiel Akarizide oder Fungizide je nach dem gewünschten Zweck zugesetzt werden.

Eine Förderung der Wirkungsintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls von Netz- Haft-, Emulgier- und/oder Dispergierhilfsmitteln angewandt.

Geeignete flüssige Trägerstoffe sind zum Beispiel Wasser, aliphatische und aromatische Kohlenwasserstoffe, weiterhin Cyclohexanon, Isophoron, Dimethylsulfoxid, Dimethylformamid und Mineralölfraktionen.

Als feste Trägerstoffe eignen sich Mineralerden, zum Beispiel Tonsil, Silikagel, Talkum, Kaolin, Attaklay, Kalkstein, Kieselsäure und pflanzliche Produkte, zum Beispiel Mehle.

An oberflächenaktiven Stoffen sind zum Beispiel zu nennen: Calciumligninsulfonat, Polyoxyäthylen-alkylphenyläther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil der bzw. des Wirkstoffe(s) in den verschiedenen Zubereitungen kann in weiten Grenzen variieren. Beispielsweise enthalten die Mittel etwa 5 bis 80 Gewichtsprozent Wirkstoffe, etwa 95 bis 20 Gewichtsprozent flüssige oder feste Trägerstoffe sowie gegebenenfalls bis zu 20 Gewichtsprozente oberflächenaktive Stoffe.



Postanschrift: Schering Aktiengesellschaft, Postfach 65 03 11, D-1000 Berlin 65 · Für Besucher: Berlin-Wedding, Müllerstraße 170-178 · Telegramme Scheringchemie Berlin
Vorstand Dr Herbert Asmis, Dr Christian Bruhn, Dr. Heinz Hannse, Horst Kramp, Dr. Klaus Pohle, Dr. Horst Witzel · Vorsitzender des Aufsichtsrats Hans-Jürgen Hamann
Sitz der Gesellschaft Berlin und Bergkamen · Handelsregister AG Charlottenburg 93 HRB 283 und AG Kamen HRB 0061 · Berliner Commerzbank AG Berlin, Konto-Nr 108700600 Bankleitzahl 100 400 00 Berliner Handels- und Frankfurter Bank, Berlin, Konto-Nr. 70045224, Bankleitzahl 100 202 00 Deutsche Bank Berlin AG, Konto-Nr 2415008 Bankleitzahl 100 700 00 Postscheckamt Berlin West, Konto-Nr 1175-101, Bankleitzahl 100 100 10

Die Ausbringung der Mittel kann in üblicher Weise erfolgen, zum Beispiel mit Wasser als Träger in Spritzbrühmengen von etwa 100 bis 3000 Liter/ha. Eine Anwendung der Mittel im sogenannten Low-Volume- und Ultra-Low-Volume-Verfahren ist ebenso möglich wie ihre Applikation in Form von sogenannten Mikrogranulaten.

Die Herstellung dieser Zubereitungen kann in an sich bekannter Art und Weise zum Beispiel durch Mahl- oder Mischverfahren, durchgeführt werden. Gewünschtenfalls können die Einzelkomponenten auch erst kurz vor ihrer Verwendung gemischt werden, wie es zum Beispiel im sogenannten Tankmixverfahren in der Praxis durchgeführt wird.

Zur Herstellung der Zubereitungen werden zum Beispiel die folgenden Bestandteile eingesetzt:

A) 20 Gewichtsprozent Wirkstoff
35 Gewichtsprozent Bentonit
8 Gewichtsprozent Calciumsalz der Ligninsulfonsäure
2 Gewichtsprozent Natriumsalz des N-Methyl-N-olein-taurins
35 Gewichtsprozent Kieselsäure

B) 45 Gewichtsprozent Wirkstoff
5 Gewichtsprozent Natriumaluminiumsilikat
15 Gewichtsprozent Cetylpolyglykoläther mit 8 Mol Äthylenoxid
2 Gewichtsprozent Spindelöl
10 Gewichtsprozent Polyäthylenglycol
23 Teile Wasser

C) 20 Gewichtsprozent Wirkstoff
75 Gewichtsprozent Isophoron
5 Gewichtsprozent Mischung aus ionischen und nichtionischen Tensiden

Die erfindungsgemäßen Verbindungen lassen sich herstellen, indem man



Postanschrift: Schering Aktiengesellschaft, Postfach 65 03 11, D-1000 Berlin 65 · Für Besucher: Berlin-Wedding, Müllerstraße 170-178 Telegramme Scheringchemie B
Vorstand Dr Herbert Asmis, Dr Christian Bruhn, Dr. Heinz Hannse, Horst Kramp, Dr. Klaus Pohle, Dr. Horst Witzel · Vorsitzender des Aufsichtsrats: Hans-Jürgen Han
Sitz der Gesellschaft Berlin und Bergkamen Handelsregister AG Charlottenburg 93 HRB 283 und AG Kamen HRB 0061 · Berliner Commerzbank AG, Berlin, Kont
108700600 Bankleitzahl 100 400 00 Berliner Handels- und Frankfurter Bank, Berlin, Konto-Nr 70045224, Bankleitzahl 100 202 00 Deutsche Bank Berlin AG, Kont
24'5008 Bankleitzahl 100 700 00 Postscheckamt Berlin West, Konto-Nr 11 75-101, Bankleitzahl 100 100 10

a) Alkoxyaniline der allgemeinen Formel

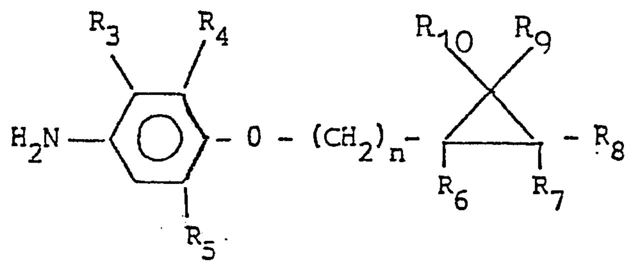

II

mit Benzoylisocyanaten oder Benzoylisothiocyanaten der allgemeinen Formel

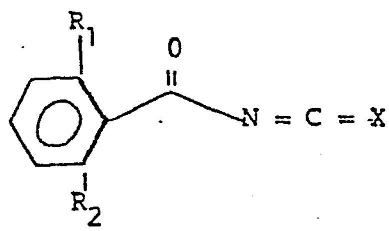

III

gegebenenfalls unter Verwendung eines Lösungsmittels sowie eines Katalysators zur Reaktion bringt oder

b) Alkoxyphenylisocyanate oder Alkoxyphenylisothiocyanate der allgemeinen Formel

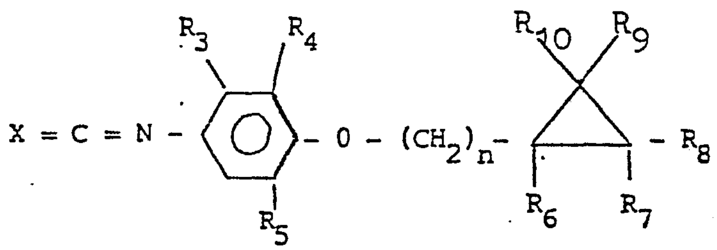

IV

mit Benzamiden der allgemeinen Formel

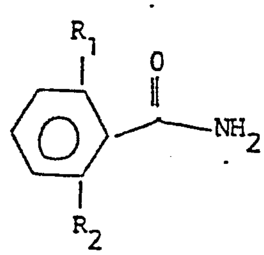

V

gegebenenfalls in Gegenwart eines Lösungsmittels sowie eines Katalysators umsetzt, wobei $R_1$, $R_2$, $R_3$, $R_4$, $R_5$, $R_6$, $R_7$, $R_8$, $R_9$, $R_{10}$, X und n die oben angegebene Bedeutung haben.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West: Konto-Nr. 1175-101, Bankleitzahl 100 100 10

Als Lösungsmittel eignen sich gegenüber den Reaktanden inerte Stoffe wie aliphatische, alicyclische und aromatische Kohlenwasserstoffe, die jeweils gegebenenfalls chloriert sein können wie Hexan, Cyclohexan, Petrolether, Benzol, Toluol, Xylol, Methylenchlorid, Trichlorethylen und Chlorbenzol; Ether, wie Diethylether, Methylethylether, Diisopropylether, Dibutylether, Propylenoxid, Dioxan und Tetrahydrofuran; Ketone, wie Aceton, Methylethylketon; Nitrile wie Acetonitril, Propionitril und Benzonitril; Ester, wie Ethylacetat und Amylacetat; Säureamide, wie Dimethylformamid und Dimethylacetamid; Sulfone und Sulfoxide, wie Dimethylsulfoxid und Sulfolan.

Die Reaktionsvariante a) kann innerhalb eines breiten Temperaturbereichs durchgeführt werden. Im allgemeinen wird sie bei einer Temperatur zwischen -20°C und dem Siedepunkt der Reaktionsmischung, vorzugsweise zwischen 20°C und 200°C durchgeführt.
Die Reaktionsvariante wird vorzugsweise unter dem Druck der Umgebung durchgeführt, wenngleich sie auch bei erhöhtem oder vermindertem Druck durchgeführt werden kann.

Die Reaktionsvariante b) kann unter den gleichen Reaktionsbedingungen in Bezug auf Temperatur und Druck durchgeführt werden, wie sie für die Reaktionsvariante a) im Vorstehenden genannt wurde.

Die erfindungsgemäßen Acylharnstoffe sind farb- und geruchlose kristalline Verbindungen. Sie lösen sich nur sehr schlecht in Wasser oder Toluol, besser in Essigester und gut in Dimethylformamid.

Die als Ausgangsmaterialien zu verwendenden Benzamide der allgemeinen Formel V und die Benzoylisocyanate und Benzoylisothiocyanate der allgemeinen Formel III sind an sich bekannt oder können nach bekannten Methoden hergestellt werden.

Die Ausgangsprodukte der allgemeinen Formel II erhält man beispielsweise nach den folgenden Herstellungsverfahren:



Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jurgen Hamann
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr 106700600 Bankleitzahl 100 400 00 · Berliner Handels- und Frankfurter Bank Berlin, Konto-Nr 70045224 Bankleitzahl 100 202 00 · Deutsche Bank Berlin AG, Konto-Nr 2415008 Bankleitzahl 100 700 00 · Postscheckamt Berlin West Konto-Nr 1175-101 Bankleitzahl 100 100 10

Aniline der allgemeinen Formel

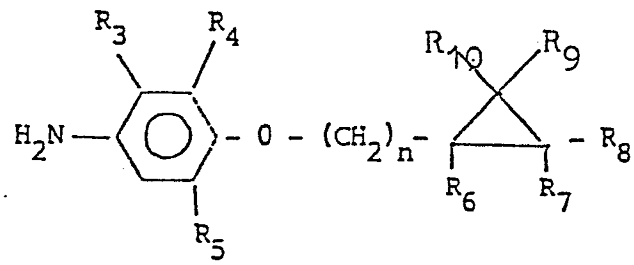

II

wobei n= 1, 2 und 3 bedeutet,
lassen sich herstellen, indem man Nitrophenole der allgemeinen Formel

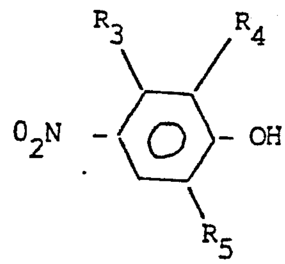

VI

mit Alkenylhalogeniden der allgemeinen Formel

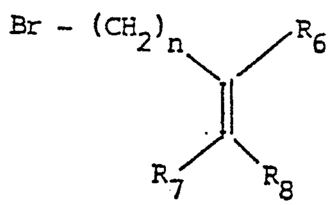

VII

verethert. Die Umsetzung mit Difluorcarben und die anschließende Reduktion nach gängigen Verfahren liefert das gewünschte Anilin.

Aniline der allgemeinen Formel II, wobei n = 0 bedeutet, lassen sich herstellen, indem man Nitrophenole der allgemeinen Formel VI mit Essigsäurevinylester in Gegenwart katalytischer Mengen Quecksilberacetat und Säuren wie zum Beispiel konz. $H_2SO_4$, p-Toluolsulfonsäure oder $BF_3$-Etherat zu Vinylethern umsetzt. Anschließend wird mit Difluorcarben umgesetzt und durch Reduktion in das gewünschte Anilin überführt.
Die angeführten Vinylether lassen sich auch in einer Zweistufenreaktion herstellen, indem man die Phenole zunächst mit 1,2-Dibromethan in Gegenwart einer schwachen Base wie zum Beispiel Kaliumcarbonat zum Bromethylether umsetzt und diesen mit einer starken Base wie zum Beispiel Kaliumhydroxid oder Kalium-tert-butylat zum Vinylether dehydrobromiert.



Postanschrift: Schering Aktiengesellschaft, Postfach 65 03 11, D-1000 Berlin 65 · Fur Besucher: Berlin-Wedding, Mullerstraße 170-178 Telegramme Scheringchemie Berlin
Vorstand: Dr Herbert Asmis, Dr Christian Bruhn, Dr Heinz Hannse, Horst Kramp, Dr. Klaus Pohle, Dr Horst Witzel Vorsitzender des Aufsichtsrats: Hans-Jurgen Hamann
Sitz der Gesellschaft: Berlin und Bergkamen Handelsregister: AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank, Berlin, Konto-Nr. 70045224, Bankleitzahl 100 202 00 Deutsche Bank Berlin AG Konto-Nr 2415008 Bankleitzahl 100 700 00 Postscheckamt Berlin West Konto-Nr 1175-101, Bankleitzahl 100 100 10

Besonders geeignet für die letzte Umsetzung sind bicyclische Amidine wie 1,5-Diazabicyclo[4.3.0]non-5-en (DBN) und 1,8-Diazabicyclo[5.4.0]undec-7-en (DBU) und tert.-Amine wie Triethylamin und 1,4-Diazabicyclo[2.2.2]-octan (DABCO).

Aniline der allgemeinen Formel II, wobei n = 1, 2 und 3 bedeutet, können auch durch direkte Veretherung von Aminophenolen hergestellt werden, indem man Aminophenole der allgemeinen Formel

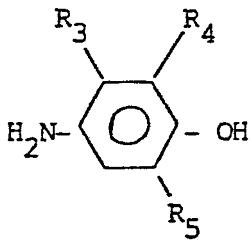

VIII

mit Difluorcyclopropylalkylbromiden der allgemeinen Formel

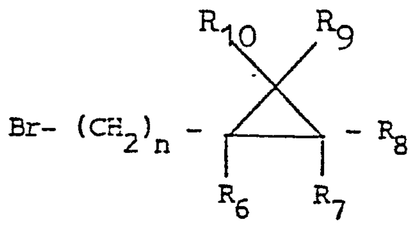

IX

verethert.

Die folgenden Beispiele erläutern die Herstellung der erfindungsgemäßen Verbindungen.

- 9 -

BEISPIEL 1

1-(2,6-Difluorbenzoyl)-3-[4-(2,2-difluorcyclopropyloxy)-phenyl]-harnstoff

1,0 g (5,4 mMol) 4-(2,2-Difluorcyclopropyloxy)-anilin werden in 10 ml THF gelöst und bei Raumtemperatur mit 988 mg (5,4 mMol) 2,6-Difluorbenzoylisocyanat, in 10 ml THF gelöst, versetzt.
Nach einer Stunde fügt man 150 ml Pentan hinzu und saugt den Niederschlag ab. Es wird mit Pentan nachgewaschen und bei 50°C im Vakuum (200 Torr) getrocknet.

| | | | | | |
|---|---|---|---|---|---|
| Ausbeute: | 1,8 g (92 % der Theorie) | | | | |
| Fp.: | 194 - 195°C | | | | |
| DC.: | Laufmittel: $CH_2Cl_2$ : MeOH = 95 : 5 | | | | |
| | $R_f$-Wert: 0,71 | | | | |
| Analyse: | Berechnet: | C 55,44 | H 3,29 | N 7,61 | F 20,64 |
| | Gefunden : | C 55,06 | H 3,33 | N 7,28 | F 20,52 |



Postanschrift: Schering Aktiengesellschaft, Postfach 65 03 11, D-1000 Berlin 65 · Fur Besucher Berlin-Wedding, Mullerstraße 170-178 Telegramme Scheringchemie Berlin
Vorstand Dr. Herbert Asmis, Dr. Christian Bruhn, Dr. Heinz Hannse, Horst Kramp, Dr. Klaus Pohle, Dr. Horst Witzel · Vorsitzender des Aufsichtsrats Hans-Jürgen Hamann
Sitz der Gesellschaft Berlin und Bergkamen · Handelsregister: AG Charlottenburg 93 HRB 283 und AG Kamen HRB 0061 Berliner Commerzbank AG, Berlin Konto-Nr 1087008600 Bankleitzahl 100 400 00 Berliner Handels- und Frankfurter Bank, Berlin Konto-Nr 70045224, Bankleitzahl 100 202 00 Deutsche Bank Berlin AG Konto-Nr 2415008 Bankleitzahl 100 700 00 Postscheckamt Berlin West Konto-Nr 1175-101, Bankleitzahl 100 100 10

BEISPIEL 2

1-(2-Chlorbenzoyl)-3-[4-(2,2-difluorcyclopropylmethoxy)-3,5-dimethylphenyl]-harnstoff

2,1 g (9,2 mMol) 4-(2,2-Difluorcyclopropylmethoxy)-3,5-dimethylanilin werden in 10 ml Tetrahydrofuran gelöst und bei Raumtemperatur mit 1,82 g (10,2 mMol) 2-Chlorbenzoylisocyanat, in 10 ml Tetrahydrofuran gelöst, versetzt.
Nach einer Stunde fügt man 150 ml Pentan hinzu und saugt den Niederschlag ab. Es wird mit Pentan nachgewaschen und bei 50°C im Vakuum (200 Torr) getrocknet.

Ausbeute: 2,9 g = 77% der Theorie
Fp.: 164-165°C
DC : Laufmittel: $CH_2Cl_2$: MeOH = 95:5
$R_f$-Wert: 0,81

| | | | | | | |
|---|---|---|---|---|---|---|
| Analyse: | Berechnet: | C 58,75 | H 4,68 | N 6,85 | Cl 8,67 | F 9,29 % |
| | Gefunden: | C 58,57 | H 4,78 | N 6,40 | Cl 8,30 | F 9,36 % |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr. 1175-101, Bankleitzahl 100 100 10

BEISPIEL 3

1-[4-(1-Chlor-2,2-difluorcyclopropylmethoxy)-3,5-dimethylphenyl]-3-(2,6-difluorbenzoyl)-harnstoff

1,25 g (4,8 m Mol) 4-(1-Chlor-2,2-difluorcyclopropylmethoxy)-3,5-dimethylanilin werden in 10 ml Tetrahydrofuran (THF) gelöst und bei Raumtemperatur mit 0,87 g (4,8 m Mol) 2,6-Difluorbenzoylisocyanat, in 10 ml THF gelöst, versetzt. Nach einer Stunde fügt man 150 ml Pentan hinzu und saugt den Niederschlag ab. Es wird mit Pentan nachgewaschen und bei 50 °C im Vakuum (200 Torr) getrocknet.

Ausbeute: 1,55 g = 73 % der Theorie
Fp.: 176-177° C
DC: Laufmittel: $CH_2Cl_2$:MeOH = 95:5
$R_f$-Wert: 0,74

| | | | | | | |
|---|---|---|---|---|---|---|
| Analyse: | Berechnet: | C 53,99 | H 3,85 | N 6,29 | Cl 7,97 | F 17,08 |
| | Gefunden: | C 53,95 | H 3,88 | N 6,06 | Cl 8,03 | F 16,85 |



Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr. 1175-101, Bankleitzahl 100 100 10

In analoger Weise wurden auch die folgenden Verbindungen hergestellt:

| Beispiel Nr. | Name der Verbindung | Physikalische Konstante |
|---|---|---|
| 4 | 1-(2,6-Difluorbenzoyl)-3-[4-(2,2-difluorcyclopropyloxy)-3,5-dimethylphenyl]-harnstoff | Fp.:184-185°C |
| 5 | 1-(2-Chlorbenzoyl)-3-[4-(2,2-difluorcyclopropyloxy)-3,5-dimethylphenyl]-harnstoff | Fp.:201-202°C |
| 6 | 1-[3,5-Dichlor-4-(2,2-difluorcyclopropyloxy)-phenyl]-3-(2,6-difluorbenzoyl)-harnstoff | Fp.:184-185°C |
| 7 | 1-[3,5-Dichlor-4-(2,2-difluorcyclopropyloxy)-phenyl]-3-(2-chlorbenzoyl)-harnstoff | Fp.:211-212°C |
| 8 | 1-(2-Chlor-6-fluorbenzoyl)-3-[4-(2,2-difluorcyclopropyloxy)-3,5-dimethyl-phenyl]-harnstoff | Fp.: 232°C |
| 9 | 1-(2,6-Dichlorbenzoyl)-3-[4-(2,2-difluorcyclopropyloxy)-3,5-dimethylphenyl]-harnstoff | Fp.: 240°C |
| 10 | 1-[4-(2,2-Difluorcyclopropyloxy)-3,5-dimethylphenyl]-3-(2-fluorbenzoyl)-harnstoff | Fp.: 123°C |
| 11 | 1-[4-(2,2-Difluorcyclopropyloxy)-3,5-dimethylphenyl]-3-(2-methylbenzoyl)-harnstoff | Fp.: 178°C |
| 12 | 1-(2-Brombenzoyl)-3-[4-(2,2-difluorcyclopropyloxy)-3,5-dimethylphenyl]-harnstoff | Fp.: 229°C |
| 13 | 1-[3-Chlor-4-(2,2-difluorcyclopropyloxy)-phenyl]-3-(2,6-difluorbenzoyl)-harnstoff | Fp.:171-172°C |
| 14 | 1-[3-Chlor-4-(2,2-difluorcyclopropyloxy)-phenyl]-3-(2-chlorbenzoyl)-harnstoff | Fp.:166-167°C |
| 15 | 1-[3-Chlor-4-(2,2-difluorcyclopropyloxy)-phenyl]-3-(2-fluorbenzoyl)-harnstoff | Fp.:171-172°C |
| 16 | 1-[3-Chlor-4-(2,2-difluorcyclopropyloxy)-phenyl]-3-(2-chlor-6-fluorbenzoyl)-harnstoff | Fp.:181-182°C |
| 17 | 1-(2,6-Difluorbenzoyl)-3-[4-(2,2-difluorcyclopropyloxy)-3-methylphenyl]-harnstoff | Fp.: 161°C |

- 13

Postanschrift: Schering Aktiengesellschaft, Postfach 65 03 11, D-1000 Berlin 65 · Fur Besucher: Berlin-Wedding, Mullerstraße 170-178 · Telegramme Scheringchemie B
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jurgen Han
Sitz der Gesellschaft: Berlin und Bergkamen · Handelsregister: AG Charlottenburg 93 HRB 283 und AG Kamen HRB 0061 · Berliner Commerzbank AG, Berlin, Kont 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Kont 2415008 Bankleitzahl 100 700 00 · Postscheckamt Berlin West Konto-Nr 1175-101 Bankleitzahl 100 100 10

| Beispiel Nr. | Name der Verbindung | Physikalische Konstante |
|---|---|---|
| 18 | 1-(2-Chlorbenzoyl)-3-[4-(2,2-difluorcyclopropyloxy)-3-methylphenyl]-harnstoff | Fp.: 181°C |
| 19 | 1-(2-Chlor-6-fluorbenzoyl)-3-[4-(2,2-difluorcyclopropyloxy)-3-methylphenyl]-harnstoff | Fp.: 184°C |
| 20 | 1-(4-(2,2-Difluorcyclopropyloxy)-3-methylphenyl]-3-(2-fluorbenzoyl)-harnstoff | Fp.: 140°C |
| 21 | 1-/3,5-Dibrom-4-(2,2-difluorcyclopropyloxy)-phenyl7-3-(2,6-difluorobenzoyl)-harnstoff | Fp.: 205-206°C |
| 22 | 1-(2,6-Difluorbenzoyl)-3-{4-/2-(2,2-difluorcyclopropyl)-ethoxy7-3,5-dimethylphenyl}-harnstoff | Fp.: 174-174°C |
| 23 | 1-(2-Chlorbenzoyl)-3-{4-/2-(2,2-difluorcyclopropyl)-ethoxy7-3,5-dimethylphenyl}-harnstoff | Fp.: 144-145°C |
| 24 | 1-{3,5-Dichlor-4-/2-(2,2-difluorcyclopropyl)-ethoxy7-phenyl}-3-(2,6-difluorbenzoyl)-harnstoff | Fp.: 167-168°C |
| 25 | 1-{3,5-Dichlor-4-/2-(2,2-difluorcyclopropyl)-ethoxy7-phenyl}-3-(2-chlorbenzoyl)-harnstoff | Fp.: 190-191°C |
| 26 | 1-(2,6-Difluorbenzoyl)-3-/4-(2,2-difluorcyclopropoxy)-3,5-dimethylphenyl7-thioharnstoff | Fp.: 144°C |
| 27 | 1-(2,6-Difluorbenzoyl)-3-/4-(2,2-difluorcyclopropoxy)-phenyl7-thioharnstoff | Fp.: 171-172°C |
| 28 | 1-/4-(2,2-Difluorcyclopropyloxy)-phenyl7-3-(2-methylbenzoyl)-harnstoff | Fp.: 172-173°C |
| 29 | 1-(2,6-Difluorbenzoyl)-3-{4-[3-(2,2--difluorcyclopropyl)-propoxy]-3,5-dimethylphenyl}-harnstoff | Fp.:154-155°C |
| 30 | 1-(2-Chlorbenzoyl)-3-{4-[3-(2,2-difluorcyclopropyl)-propoxy]-3,5-dimethylphenyl}-harnstoff | Fp.:146-147°C |

- 14 -

| Beispiel Nr. | Name der Verbindungen | Physikalische Konstante |
|---|---|---|
| 31 | 1-{3,5-Dichlor-4-[3-(2,2-difluorcyclopropyl)-propoxy]-phenyl}-3-(2,6-difluorbenzoyl)-harnstoff | Fp.:158-159° C |
| 32 | 1-{3,5-Dichlor-4-[3-(2,2-difluorcyclopropyl)-propoxy]-phenyl}-3-(2-chlorbenzoyl)-harnstoff | Fp.:179-180° C |
| 33 | 1-{4-[3-(2,2-Difluorcyclopropyl)-propoxy]-3,5-dimethylphenyl}-3-(2-fluorbenzoyl)-harnstoff | Fp.:117-118° C |
| 34 | 1-{3,5-Dichlor-4-[3-(2,2-difluorcyclopropyl)-propoxy]-phenyl}-3-(2-fluorbenzoyl)-harnstoff | Fp.:139-140° C |
| 35 | 1-{4-[2-(2,2-Difluorcyclopropyl)-ethoxy]-3,5-dimethylphenyl}-3-(2-fluorbenzoyl)-harnstoff | Fp.:140-141° C |
| 36 | 1-[3,5-Dichlor-4-(2,2-difluorcyclopropyloxy)-phenyl]-3-(2-fluorbenzoyl)-harnstoff | Fp.:167-168°C |
| 37 | 1-{3,5-Dichlor-4-[2-(2,2-difluorcyclopropyl)-ethoxy]-phenyl}-3-(2-fluorbenzoyl)-harnstoff | Fp.:164-165°C |
| 38 | 1-(2,6-Difluorbenzoyl)-3-[4-(2,2-difluorcyclopropylmethoxy)-phenyl]-harnstoff | Fp.: 179°C |
| 39 | 1-(2-Chlorbenzoyl)-3-[4-(2,2-difluorcyclopropylmethoxy)-phenyl]-harnstoff | Fp.: 165°C |
| 40 | 1-(2,6-Difluorbenzoyl)-3-[4-(2,2-difluorcyclopropylmethoxy)-3,5-dimethylphenyl]-harnstoff | Fp.: 152°C |
| 41 | 1-[3,5-Dichlor-4-(2,2-difluorcyclopropylmethoxy)-phenyl]-3-(2,6-difluorbenzoyl)-harnstoff | Fp.: 170-171°C |
| 42 | 1-[3,5-Dichlor-4-(2,2-difluorcyclopropylmethoxy)-phenyl]-3-(2-chlorbenzoyl)-harnstoff | Fp.: 193-194°C |

- 15

| Beispiel Nr. | Name der Verbindungen | Physikalische Konstante |
|---|---|---|
| 43 | 1-[3-Chlor-4-(2,2-difluorcyclopropylmethoxy)-phenyl]-3-(2,6-difluorbenzoyl)-harnstoff | Fp.: 169–170°C |
| 44 | 1-[3-Chlor-4-(2,2-difluorcyclopropylmethoxy)-phenyl]-3-(2-chlorbenzoyl)-harnstoff | Fp.: 159–160°C |
| 45 | 1-[3-Chlor-4-(2,2-difluorcyclopropylmethoxy)-phenyl]-3-(2-fluorbenzoyl)-harnstoff | Fp.: 164–165°C |
| 46 | 1-[3-Chlor-4-(2,2-difluorcyclopropylmethoxy)-phenyl]-3-(2-chlor-6-fluorbenzoyl)-harnstoff | Fp.: 182–183°C |
| 47 | 1-(2,6-Difluorbenzoyl)-3-[4-(2,2-difluorcyclopropylmethoxy)-3-methylphenyl]-harnstoff | Fp.: 162–163°C |
| 48 | 1-(2-Chlorbenzoyl)-3-[4-(2,2-difluorcyclopropylmethoxy)-3-methylphenyl]-harnstoff | Fp.: 151–152°C |
| 49 | 1-(2-Chlor-6-fluorbenzoyl)-3-[4-(2,2-difluorcyclopropylmethoxy)-3-methyl-phenyl]-harnstoff | Fp.: 151–152°C |
| 50 | 1-[4-(2,2-Difluorcyclopropylmethoxy)-3-methylphenyl]-3-(2-fluorbenzoyl)-harnstoff | Fp.: 131–132°C |
| 51 | 1-[3,5-Dibrom-4-(2,2-difluorcyclopropylmethoxy)-phenyl]-3-(2,6-difluorbenzoyl)-harnstoff | Fp.: 166–167°C |
| 52 | 1-[3,5-Dibrom-4-(2,2-difluorcyclopropylmethoxy)-phenyl]-3-(2-chlorbenzoyl)-harnstoff | Fp.: 191–192°C |
| 53 | 1-[3,5-Dibrom-4-(2,2-difluorcyclopropylmethoxy)-phenyl]-3-(2,6-dichlorbenzoyl)-harnstoff | Fp.: 209–210°C |
| 54 | 1-[3,5-Dibrom-4-(2,2-difluorcyclopropylmethoxy)-phenyl]-3-(2-brombenzoyl)-harnstoff | Fp.: 182–183°C |

- 16 -

| Beispiel Nr. | Name der Verbindungen | Physikalische Konstante |
|---|---|---|
| 55 | 1-(2,6-Difluorbenzoyl)-3-[4-(2,2-difluorcyclopropylmethoxy)-3,5-dijodphenyl]-harnstoff | Fp.: 204-205°C |
| 56 | 1-(2,6-Difluorbenzoyl)-3-[4-(2,2-difluor-1-methylcyclopropylmethoxy)-3,5-dimethylphenyl]-harnstoff | Fp.: 186°C |
| 57 | 1-(2,6-Difluorbenzoyl)-3-[4-(2,2-difluor-3-methylcyclopropylmethoxy)-3,5-dimethylphenyl]-harnstoff | Fp.: 151°C |
| 58 | 1-(2,6-Difluorbenzoyl)-3-[4-(2,2-difluor-3-phenylcyclopropylmethoxy)-3,5- dimethylphenyl]-harnstoff | Fp.: 120-121°C |
| 59 | 1-(2-Chlorbenzoyl)-3-[4-(2,2-difluor-1-methylcyclopropylmethoxy)-3,5-dimethylphenyl]-harnstoff | Fp.: 198°C |
| 60 | 1-(2-Chlorbenzoyl)-3-[4-(2,2-difluor-3-phenylcyclopropylmethoxy)-3,5-dimethylphenyl]-harnstoff | Fp.: 112-113°C |
| 61 | 1-(2-Chlorbenzoyl)-3-[4-(2,2-difluor-3-methylcyclopropylmethoxy)-3,5-dimethylphenyl]-harnstoff | Fp.: 156°C |
| 62 | 1-[3,5-Dichlor-4-(2,2-difluor-1-methylcyclopropylmethoxy)-phenyl]-3-(2,6-difluorbenzoyl)-harnstoff | Fp.: 180°C |
| 63 | 1-[3,5-Dichlor-4-(2,2-difluor-3-methylcyclopropylmethoxy)-phenyl]-3-(2,6-difluorbenzoyl)-harnstoff | Fp.: 140°C |
| 64 | 1-[3,5-Dichlor-4-(2,2-difluor-3-phenylcyclopropylmethoxy)-phenyl]-3-(2,6-difluorbenzoyl)-harnstoff | Fp.: 145-146°C |
| 65 | 1-[3,5-Dichlor-4-(2,2-difluor-1-methylcyclopropylmethoxy)-phenyl]-3-(2-chlorbenzoyl)-harnstoff | Fp.: 220°C |

- 17

Postanschrift: Schering Aktiengesellschaft, Postfach 65 03 11, D-1000 Berlin 65 · Für Besucher: Berlin-Wedding, Müllerstraße 170-178 Telegramme: Scheringchemie
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Ha
Sitz der Gesellschaft: Berlin und Bergkamen · Handelsregister: AG Charlottenburg 93 HRB 283 und AG Kamen HRB 0061 · Berliner Commerzbank AG, Berlin, Kor
108790600 Bankleitzahl 100 400 00 · Berliner Handels- und Frankfurter Bank, Berlin, Konto-Nr. 70045224, Bankleitzahl 100 202 00 · Deutsche Bank Berlin AG, Kor
2415008 Bankleitzahl 100 700 00 · Postscheckamt Berlin West, Konto-Nr. 1175-101, Bankleitzahl 100 100 10

| Beispiel Nr. | Name der Verbindungen | Physikalische Konstante |
|---|---|---|
| 66 | 1-[3,5-Dichlor-4-(2,2-difluor-3-methylcyclopropylmethoxy)-phenyl]-3-(2-chlorbenzoyl)-harnstoff | Fp.: 183°C |
| 67 | 1-[3,5-Dichlor-4-(2,2-difluor-3-phenylcyclopropylmethoxy)-phenyl]-3-(2-chlorbenzoyl)-harnstoff | Fp.: 147-148°C |
| 68 | 1-[3,5-Dibrom-4-(2,2-difluorcyclopropylmethoxy)-phenyl]-3-(2-methylbenzoyl)-harnstoff | Fp.:191-192°C |
| 69 | 1-[3,5-Dichlor-4-(2,2-difluorcyclopropylmethoxy)-phenyl]-3-(2-methylbenzoyl)-harnstoff | Fp.:189-190°C |
| 70 | 1-[3,5-Dichlor-4-(2,2-difluor-1-methylcyclopropylmethoxy)-phenyl]-3-(2-methylbenzoyl)-harnstoff | Fp.: 210°C |
| 71 | 1-/4-(2,2-Difluorcyclopropylmethoxy)-3,5-dimethylphenyl7-3-(2-fluorbenzoyl)-harnstoff | Fp.: 110-111°C |
| 72 | 1-/3,5-Dichlor-4-(2,2-difluorcyclopropylmethoxy)-phenyl7-3-(2-fluorbenzoyl)-harnstoff | Fp.: 160-161°C |
| 73 | 1-[3,5-Dibrom-4-(2,2-difluorcyclopropylmethoxy)-phenyl]-3-(2-fluorbenzoyl)-harnstoff | Fp.:173-174°C |
| 74 | 1-[3,5-Dichlor-4-(2,2-difluor-3-methylcyclopropylmethoxy)-phenyl]-3-(2-fluorbenzoyl)-harnstoff | Fp.:148-149°C |
| 75 | 1-[3,5-Dibrom-4-(2,2-difluor-3-methylcyclopropylmethoxy)-phenyl]-3-(2-fluorbenzoyl)-harnstoff | Fp.:150-151°C |
| 76 | 1-[3,5-Dibrom-4-(2,2-difluor-1-methylcyclopropylmethoxy)-phenyl]-3-(2-fluorbenzoyl)-harnstoff | Fp.:160-161°C |
| 77 | 1-[3,5-Dibrom-4-(2,2-difluor-3-methylcyclopropylmethoxy)-phenyl]-3-(2,6-difluorbenzoyl)-harnstoff | Fp.:160-161°C |

- 18 -

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

| Beispiel Nr. | Name der Verbindungen | Physikalische Konstante |
|---|---|---|
| 78 | 1-[3,5-Dibrom-4-(2,2-difluor-3-methyl-cyclopropylmethoxy)-phenyl]-3-(2-chlor-benzoyl)-harnstoff | Fp.: 178-179 °C |
| 79 | 1-[3,5-Dibrom-4-(2,2-difluor-1-methyl-cyclopropylmethoxy)-phenyl]-3-(2,6-di-fluorbenzoyl)-harnstoff | Fp.: 188-189 °C |
| 80 | 1-[3,5-Dibrom-4-(2,2-difluor-1-methyl-cyclopropylmethoxy)-phenyl]-3-(2-chlor-benzoyl)-harnstoff | Fp.: 197-198 °C |
| 81 | 1-[4-(2,2-Difluor-1-methylcyclopropyl-methoxy)-3,5-dimethylphenyl)-3-(2-fluor-benzoyl)-harnstoff | Fp.: 162-163 °C |
| 82 | 1-(2,6-Difluorbenzoyl)-3-[4-(2,2-di-fluorcyclopropylmethoxy)-3,5-difluor-phenyl]-harnstoff | Fp.: 173-174 °C |
| 83 | 1-[4-(2,2-Difluorcyclopropylmethoxy)-3,5-difluorphenyl]-3-(2-fluorbenzoyl)-harnstoff | Fp.: 146-147 °C |
| 84 | 1-(2-Chlorbenzoyl)-3-[4-(2,2-difluor-cyclopropylmethoxy)-3,5-difluorphenyl]-harnstoff | Fp.: 149-150 °C |
| 85 | 1-[4-(1-Chlor-2,2-difluorcyclopropyl-methoxy)-3,5-dimethylphenyl]-3-(2-chlor-benzoyl)-harnstoff | Fp.: 192-193 °C |
| 86 | 1-[4-(1-Chlor-2,2-difluorcyclopropyl-methoxy)-3,5-dimethylphenyl]-3-(2-fluor-benzoyl)-harnstoff | Fp.: 145-146 °C |
| 87 | 1-[4-(1-Chlor-2,2-difluorcyclopropyl-methoxy)-3,5-dimethylphenyl]-3-(2-chlor-6-fluorbenzoyl)-harnstoff | Fp.: 204-205 °C |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Ha
Sitz der Gesellschaft: Berlin und Bergkamen · Handelsregister: AG Charlottenburg 93 HRB 283 und AG Kamen HRB 0061 · Berliner Commerzbank AG, Berlin, Kto
108700600, Bankleitzahl 100 400 00 · Berliner Handels- und Frankfurter Bank, Berlin, Konto-Nr. 70045224, Bankleitzahl 100 202 00 · Deutsche Bank Berlin AG, Kto
2415008, Bankleitzahl 100 700 00 · Postscheckamt Berlin West, Konto-Nr. 1175-101, Bankleitzahl 100 100 10

| Beispiel Nr. | Name der Verbindungen | Physikalische Konstante |
|---|---|---|
| 88 | 1-(2,6-Difluorbenzoyl)-3-/4-(2,2-difluor-3,3-dimethylcyclopropylmethoxy)-3,5-dimethylphenyl7-harnstoff | Fp.: 175°C |
| 89 | 1-(2-Chlorbenzoyl)-3-/4-(2,2-difluor-3,3-dimethylcyclopropylmethoxy)-3,5-dimethylphenyl7-harnstoff | Fp.: 172°C |
| 90 | 1-/3,5-Dichlor-4-(2,2-difluor-3,3-dimethylcyclopropylmethoxy)-phenyl7-3-(2,6-difluorbenzoyl)-harnstoff | Fp.: 170°C |
| 91 | 1-(2-Chlorbenzoyl)-3-/3,5-dichlor-4-(2,2-difluor-3,3-dimethylcyclopropylmethoxy)-phenyl7-harnstoff | Fp.: 188°C |
| 92 | 1-/4-(1-Chlor-2,2-difluorcyclopropylmethoxy)-3,5-dichlorphenyl7-3-(2,6-difluorbenzoyl)-harnstoff | Fp.: 171-172°C |
| 93 | 1-/4-(1-Chlor-2,2-difluorcyclopropylmethoxy)-3,5-dichlorphenyl7-3-(2-chlorbenzoyl)-harnstoff | Fp.: 202-203°C |
| 94 | 1-/4-(1-Chlor-2,2-difluorcyclopropylmethoxy)-3,5-dichlorphenyl7-3-(2-chlor-6-fluorbenzoyl)-harnstoff | Fp.: 182-183°C |



Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin

Vorstand Dr. Herbert Asmis Dr. Christian Bruhn, Dr. Heinz Hannse, Horst Kramp, Dr. Klaus Pohle, Dr. Horst Witzel Vorsitzender des Aufsichtsrats: Hans-Jurgen Hamann
Sitz der Gesellschaft Berlin und Bergkamen Handelsregister: AG Charlottenburg 93 HRB 283 und AG Kamen HRB 0061 Berliner Commerzbank AG, Berlin Konto-Nr 108700600, Bankleitzahl 100 400 00 Berliner Handels- und Frankfurter Bank, Berlin, Konto-Nr 70045224 Bankleitzahl 100 202 00 Deutsche Bank Berlin AG, Konto-Nr 2415008, Bankleitzahl 100 700 00 Postscheckamt Berlin West, Konto-Nr. 1175-101, Bankleitzahl 100 100 10

| Beispiel Nr. | Name der Verbindungen | Physikalische Konstante |
|---|---|---|
| 95 | 1-/4-(1-Chlor-2,2-difluorcyclopropylmethoxy)-3,5-dichlorphenyl/-3-(2-fluorbenzoyl)-harnstoff | Fp.: 160-161°C |
| 96 | 1-/4-(1-Chlor-2,2-difluorcyclopropylmethoxy)-3,5-dichlorphenyl/-3-(2,6-difluorbenzoyl)-thioharnstoff | Fp.: 147-148°C |
| 97 | 1-(3,5-Dichlor-4-cyclopropylmethoxyphenyl)-3-(2,6-difluorbenzoyl)-harnstoff | Fp.: 180-181° C |
| 98 | 1-(3,5-Dichlor-4-cyclopropylmethoxyphenyl)-3-(2-fluorbenzoyl)-harnstoff | Fp.: 159-160° C |

Die folgenden Beispiele erläutern die Herstellung der Ausgangsverbindungen der allgemeinen Formel II.



Postanschrift: Schering Aktiengesellschaft, Postfach 65 03 11, D-1000 Berlin 65 · Fur Besucher Berlin-Wedding, Mullerstraße 170-178 Telegramme Scheringchemie Berlin
Vorstand: Dr Herbert Asmis Dr Christian Bruhn, Dr Heinz Hannse, Horst Kramp, Dr. Klaus Ponte, Dr Horst Witzel Vorsitzender des Aufsichtsrats: Hans-Jurgen Hamann Sitz der Gesellschaft Berlin und Bergkamen Handelsregister AG Charlottenburg 93 HRB 283 und AG Kamen HRB 0061 Berliner Commerzbank AG, Berlin, Konto-Nr 108700600, Bankleitzahl 100 400 00 Berliner Handels- und Frankfurter Bank, Berlin, Konto-Nr 70045224, Bankleitzahl 100 202 00 · Deutsche Bank Berlin AG Konto-Nr 2415008 Bankleitzahl 100 700 00 Postscheckamt Berlin West, Konto-Nr 1175-101, Bankleitzahl 100 100 10

BEISPIEL 99

4-(2,2-Difluorcyclopropyloxy)-3,5-dimethylanilin

50 g (0,30 Mol) 4-Nitro-2,6-dimethylphenol, 45,4 g (0,33 Mol) Kaliumcarbonat und 1,5 g 18-Krone-6 versetzt man mit 250 ml 1,2-Dibromethan und rührt 8 Stunden bei 100 °C. Anschließend gibt man auf Eiswasser und extrahiert mit Chloroform. Die Chloroformphase wird mit 10%iger Natronlauge und viermal mit Wasser gewaschen, über Magnesiumsulfat getrocknet und im Vakuum eingedampft. Aus Hexan erhält man 67 g (82% der Theorie; 0,24 Mol) 2-Bromethyl-2,6-dimethyl-4-nitrophenylether (Fp.: 86-87°C).

Die gesamte Substanzmenge wird in 300 ml Toluol gelöst, mit 2 g 18-Krone-6 und 30 g (0,54 Mol) Kaliumhydroxid versetzt und 6 Stunden bei 60°C gerührt. Nach dem Abkühlen wird die Toluolphase mit Wasser gewaschen, getrocknet und im Vakuum eingeengt. Aus Hexan erhält man 31,6 g (67 % der Theorie) 2,6-Dimethyl-4-nitrophenylvinylether (Fp.: 78°C)
15 g (78 mMol) dieses Vinylethers, in 100 ml Diglym gelöst, versetzt man bei 165°C Innentemperatur innerhalb von 2 Stunden mit einer Lösung von 59,1 g (388 mMol) Natriumchloridfluoracetat in 100 ml Diglym. Man rührt 1 Stunde bei 165°C nach, läßt abkühlen, filtriert die anorganischen Bestandteile ab und engt im Vakuum (0,1 Torr; 50°C) ein. Der Rückstand, der durch Säulenchromatographie (Hexan:Toluol, 9:1) gereinigt wird, ergibt 14,6 g (77 % der Theorie) 4-(2,2-Difluorcyclopropyloxy)-3,5-dimethylnitrobenzol (Fp.: 63-64°C)

8 g (33mMol) 4-(2,2-Difluorcyclopropyloxy)-3,5-dimethylnitrobenzol suspendiert man in 45 ml Wasser und 75 ml Ethanol und fügt nach Zugabe von 3,27 g (61,8 mMol) Ammoniumchlorid innerhalb von 10 Minuten portionsweise 16,5 g (248 mMol) Zinkpulver hinzu. Dabei steigt die Temperatur auf 40-50°C an. Nach einer Stunde wird das Zinkhydroxid abgesaugt, Ethanol vorsichtig im Vakuum entfernt und der Rückstand in Essigester aufgenommen. Die wäßrige Phase wird abgetrennt und das Lösungsmittel im Vakuum verdampft. Aus Hexan erhält man farblose Kristalle, die bei Raumtemperatur (200 Torr) getrocknet werden.

| | | | | |
|---|---|---|---|---|
| Ausbeute: | 6,5 g = 93 % der Theorie | | | |
| Fp: | 78-79°C | | | |
| DC: | Laufmittel: $CH_2Cl_2$:MeOH = 95:5 | | | |
| | $R_f$-Wert: 0,61 | | | |
| Analyse: | Berechnet: C 61,96 % | H 6,14 % | N 6,56 % | F 17,82 % |
| | Gefunden : C 62,33 % | H 6,02 % | N 6,49 % | F 17,52 % |



Postanschrift: Schering Aktiengesellschaft, Postfach 65 03 11, D-1000 Berlin 65 · Fur Besucher Berlin-Wedding, Mullerstraße 170-178 Telegramme Scheringchemie Berlin
Vorstand Dr Herbert Asmis, Dr Christian Bruhn, Dr Heinz Hannse, Horst Kramp, Dr Klaus Pohle, Dr Horst Witzel Vorsitzender des Aufsichtsrats: Hans-Jurgen Hamann
Sitz der Gesellschaft Berlin und Bergkamen · Handelsregister AG Charlottenburg 93 HRB 283 und AG Kämen HRB 0061 Berliner Commerzbank AG Berlin Konto-Nr 108700600, Bankleitzahl 100 400 00 Berliner Handels- und Frankfurter Bank, Berlin, Konto-Nr 70045224, Bankleitzahl 100 202 00 Deutsche Bank Berlin AG Konto-Nr 2415008 Bankleitzahl 100 700 00 Postscheckamt Berlin West, Konto-Nr 1175-101, Bankleitzahl 100 100 10

BEISPIEL 100

4-(2,2-Difluorcyclopropylmethoxy)-3,5-dimethylanilin

Zu 50 g (0,30 Mol) 2,6-Dimethyl-4-nitrophenol, 55,6 g (0,40 Mol) Kaliumcarbonat und 7,5 g Kaliumjodid in 400 ml Dimethylformamid addiert man bei Raumtemperatur 28,3 ml (0,33 Mol) Allylbromid. Man rührt 1,5 h bei 70°C, läßt abkühlen und filtriert die anorganischen Bestandteile ab. Anschließend verdampft man im Vakuum, versetzt mit Essigester und wäscht die organische Phase mit Wasser.
Nach Einengen und Trocknen (Raumtemperatur; 0,1 torr) erhält man 61,9 g (100%) kristallinen 2,6-Dimethyl-4-nitrophenyl-3-propenylether; Fp.: 35°C

20 g (96,5 mMol) dieses Allylethers, in 120 ml Diglym gelöst, versetzt man bei 165°C Innentemperatur innerhalb von 2 Stunden mit einer Lösung von 73,6 g (482 mMol) Natriumchlordifluoracetat in 150 ml Diglym. Man rührt 1 Stunde bei 165°C nach, läßt abkühlen, filtriert die anorganischen Bestandteile ab und engt im Vakuum (0,1 Torr; 50°C) ein. Der Rückstand, der durch Säulenchromatographie (Hexan:Toluol, 9:1) gereinigt wird, ergibt 6,3 g (26% der Theorie) 4-(2,2-Difluorcyclopropylmethoxy)-3,5-dimethylnitrobenzol ($n_D20$ = 1,5198) und 7 g (35%) Ausgangsmaterial.

5,04 g (19,6 mMol) 4-(2,2-Difluorcyclopropylmethoxy)-3,5-dimethylnitrobenzol suspendiert man in 30 ml Wasser und 50 ml Ethanol und fügt nach Zugabe von 1,91 g (36,7 mMol) Ammoniumchlorid innerhalb von 10 Minuten portionsweise 9,77 g (147 mMol) Zinkpulver hinzu. Dabei steigt die Temperatur auf 40 bis 50°C an. Nach einer Stunde wird das Zinkhydroxid abgesaugt, Ethanol vorsichtig im Vakuum entfernt und der Rückstand in Essigester aufgenommen. Die wäßrige Phase wird abgetrennt und das Lösungsmittel im Vakuum verdampft. Aus Hexan erhält man farblose Kristalle, die bei Raumtemperatur (200 Torr) getrocknet werden.

| | | | | | |
|---|---|---|---|---|---|
| Ausbeute: | 3,5 g = 79% der Theorie | | Fp.: 71-72°C | | |
| DC: | Laufmittel: $CH_2Cl_2$:MeOH = 95:5; | | Rf-Wert: 0,73 | | |
| Analyse: | Berechnet: C 63,41 % | H 6,65 % | N 6,16 % | F 16,72 % | |
| | Gefunden : C 63,29 % | H 6,70 % | N 5,91 % | F 16,76 % | |

- 23 -

Postanschrift: Schering Aktiengesellschaft, Postfach 65 03 11, D-1000 Berlin 65 · Für Besucher Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224 Bankleitzahl 100 202 00 · Deutsche Bank Berlin AG, Konto-Nr. 2415008 Bankleitzahl 100 700 00 · Postscheckamt Berlin West Konto-Nr. 1175-101, Bankleitzahl 100 100 10

BEISPIEL 101

3,5-Dichlor-4-(2,2-difluorcyclopropylmethoxy)-anilin

Zu einer Suspension von 4,16 g (23,4 mMol) 4-Amino-2,6-dichlorphenol, 3,54 g (25,7 mMol) Kaliumcarbonat und 0,74 g Kaliumjodid in Dimethylformamid (50 ml) tropft man bei 100°C (Badtemperatur) 4,0 g (23,4 mMol) 2,2-Difluorcyclopropylmethylbromid gelöst in 15 ml Dimethylformamid.
Nach einer Stunde wird im Vakuum eingeengt. Der Rückstand wird mit Essigester aufgenommen und mit Wasser gewaschen (2x). Nach dem Trocknen über Magnesiumsulfat versetzt man mit Pentan und saugt die Kristalle ab.

| | | | | | |
|---|---|---|---|---|---|
| Ausbeute: | 2,9 g = 46% der Theorie | | | | |
| Fp.: | 89 - 90°C | | | | |
| DC: | Laufmittel: $CH_2Cl_2$:MeOH = 95:5 | | | | |
| | $R_f$-Wert: 0,61 | | | | |
| Analyse: | Berechnet: | C 44,79% | H 3,38% | N 5,22% | F 14,17% |
| | Gefunden: | C 44,51% | H 3,70% | N 5,03% | F 14,32% |



Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann Sitz der Gesellschaft: Berlin und Bergkamen Handelsregister: AG Charlottenburg 93 HRB 283 und AG Kamen HRB 0061 Berliner Commerzbank AG, Berlin, Konto-Nr 108700600 Bankleitzahl 100 400 00 Berliner Handels- und Frankfurter Bank, Berlin, Konto-Nr 70045224, Bankleitzahl 100 202 00 Deutsche Bank Berlin AG Konto-Nr 2415008 Bankleitzahl 100 700 00 Postscheckamt Berlin West, Konto-Nr. 1175-101 Bankleitzahl 100 100 10

## BEISPIEL 102

4-(1-Chlor-2,2-difluorcyclopropylmethoxy)-3,5-dichloranilin

Zu 20 g (96,8 mMol) 2,6-Dichlor-4-nitrophenol, 17,9 g (0,13 Mol) Kaliumcarbonat und 2,4 g Kaliumjodid in 200 ml Dimethylformamid addiert man bei Raumtemperatur 11,8 g (0,1 Mol) 2,3-Dichlor-1-propen. Man rührt 1,5 h bei 70 °C, läßt abkühlen und filtriert die organischen Bestandteile ab. Anschließend verdampft man im Vakuum, versetzt mit Essigester und wäscht die organische Phase mit Wasser. Nach Einengen, Umkristallisation aus Isopropanol und Trocknen (Raumtemperatur; 0,1 Torr) erhält man 12,0 g (44 % d. Th.) kristallines 4-(2-Chlor-2-propenyloxy)-3,5-dichlornitrobenzol (Fp.: 53-54 °C).

12 g (42,5 mMol) dieses Phenolethers, in 150 ml Diglym gelöst, versetzt man bei 165 °C Innentemperatur innerhalb von 2 Stunden mit einer Lösung von 32,4 g (212 mMol) Natriumchlordifluoracetat in 150 ml Diglym. Man rührt 1 Stunde bei 165 °C nach, läßt abkühlen, filtriert die anorganischen Bestandteile ab und engt im Vakuum (0,1 Torr; 50 °C) ein. Der Rückstand, der durch Säulenchromatographie (Hexan:Toluol, 9:1) gereinigt wird, ergibt 10,5 g (74,8 % d. Th.) 4-(1-Chlor-2,2-difluorcyclopropylmethoxy)-3,5-dichlornitrobenzol (Fp.: 44-45 °C).

7,1 g (21,4 mMol) 4-(1-Chlor-2,2-difluorcyclopropylmethoxy)-3,5-dichlornitrobenzol suspendiert man in 35 ml Wasser und 50 ml Ethanol und fügt nach Zugabe von 2 g (40 mMol) Ammoniumchlorid innerhalb von 10 Minuten portionsweise 10,65 g (160 mMol) Zinkpulver hinzu. Dabei steigt die Temperatur auf 40-50 °C an. Nach einer Stunde wird das Zinkhydroxid abgesaugt, Ethanol vorsichtig im Vakuum ent-

- 25 -

Postanschrift: Schering Aktiengesellschaft, Postfach 65 03 11, D-1000 Berlin 65 · Für Besucher Berlin-Wedding, Müllerstraße 170-178 Telegramme Scheringchemie

Vorstand Dr Herbert Asmis Dr Christian Brunn Dr Heinz Hannse, Horst Kramp Dr Klaus Pohle Dr Horst Witzel Vorsitzender des Aufsichtsrats Hans-Jürgen H
Sitz der Gesellschaft Berlin und Bergkamen Handelsregister AG Charlottenburg 93 HRB 283 und AG Kamen HRB 0061 Berliner Commerzbank AG, Berlin Ko
108700600, Bankleitzahl 100 400 00 Berliner Handels- und Frankfurter Bank Berlin Konto-Nr 70045224, Bankleitzahl 100 202 00 Deutsche Bank Berlin AG Ko
2415008, Bankleitzahl 100 700 00 Postscheckamt Berlin West, Konto-Nr 11 75-101, Bankleitzahl 100 100 10

fernt und der Rückstand in Essigester aufgenommen. Die wäßrige Phase wird abgetrennt und das Lösungsmittel im Vakuum verdampft. Man erhält ein farbloses Öl, das nach dem Trocknen (0,1 Torr; RT) gleich weiter eingesetzt wird.

Ausbeute: 6,3 g = 97 % der Theorie

DC: Laufmittel: Hexan/Essigester 8:2
$R_f$-Wert: 0,30

$^1$H-NMR: ($CDCl_3$; TMS; 80 MHz)
s 6,52 ppm (2H, Ar-H)
$s_{br.}$ 4,17 ppm (2H, $-O-CH_2-$)
$s_{br.}$ 3,5 ppm (2H, $-NH_2$)
m 1.6-2.4 ppm (2H, 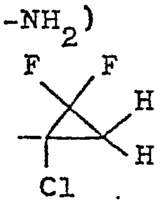 )



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand: Dr. Herbert Asmis, Dr. Christian Brunn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG Berlin Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG Konto-Nr. 2415008, Bankleitzahl 100 700 00 Postscheckamt Berlin West Konto-Nr. 1175-101, Bankleitzahl 100 100 10

BEISPIEL 103

4-(2,2-Difluor-3,3-dimethylcyclopropylmethoxy)-3,5-dichloranilin

Zu einer Suspension von 4,45 g (25 mMol) 4-Amino-2,6-dichlorphenol, 4,54 g (33 mMol) Kaliumcarbonat und 0,5 g Kaliumjodid in 50 ml Dimethylformamid tropft man bei 100 °C (Badtemperatur) 5,0 g (25 mMol) 2,2-Difluor-3,3-dimethylcyclopropylmethylbromid gelöst in 15 ml Dimethylformamid. Nach einer Stunde wird im Vakuum eingeengt. Der Rückstand wird mit Essigester aufgenommen und mit Wasser gewaschen (2mal). Nach dem Trocknen liefert die Säulenchromatographie (Hexan/Essigester, 7:3) ein farbloses Öl.

Ausbeute: 4,1 g = 55 % der Theorie

DC: Laufmittel = Hexan/Essigester 1:1
$R_f$-Wert: 0,79.

| | | | | | |
|---|---|---|---|---|---|
| Analyse: | Berechnet: | C 48,67% | H 4,42% | N 4,73% | F 12,83% |
| | Gefunden: | C 48.46% | H 4,40% | N 4,43% | F 12,65% |

- 27

Postanschrift: Schering Aktiengesellschaft, Postfach 65 03 11, D-1000 Berlin 65 · Für Besucher Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin Konto 108700600, Bankleitzahl 100 400 00 · Berliner Handels- und Frankfurter Bank Berlin Konto-Nr. 70045224, Bankleitzahl 100 202 00 · Deutsche Bank Berlin AG Konto 2415006, Bankleitzahl 100 700 00 · Postscheckamt Berlin West Konto-Nr. 1175-101, Bankleitzahl 100 100 10

Die folgenden Beispiele dienen zur Erläuterung der Anwendungsmöglichkeiten der erfindungsgemäßen Verbindungen, die in Form ihrer Zubereitungen erfolgte.

## BEISPIEL 104

Abtötende Wirkung auf Larven (L2) der Ägyptischen Baumwolleule (Spodoptera littoralis)

Die erfindungsgemäßen Substanzen wurden als wäßrige Emulsionen mit der Wirkstoffkonzentration 0,1 % bzw. 0,01 % eingesetzt.

In diese Wirkstoffzubereitungen wurden Fiederblätter der Puffbohne (Vicia faba) getaucht. Nach dem Antrocknen der Präparatebrühe auf den getauchten Fiederblättern wurden je Versuchsglied 10 Raupen der Ägyptischen Baumwoll-Eule (Spodoptera littoralis) im 2. Larvenstadium zusammen mit drei Fiederblattpaaren in einer Glaspetrischale (Ø 12 cm) eingeschlossen. Diese Versuchsglieder wurden für 5 Tage unter Langtagbedingungen im Labor aufgestellt.

Kriterium für die Wirkungsbeurteilung war die Mortalität der Raupen in % nach 5-tägiger Versuchsdauer.

- 28 -

Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175-101 Bankleitzahl 100 100 10

| Erfindungsgemäße Verbindungen | Wirkstoffkonzentration in % | Wirkung in % |
|---|---|---|
| 1-(2,6-Difluorbenzoyl)-3-[4-(2,2-difluorcyclopropyloxy)-phenyl]-harnstoff | 0,1 | 100 |
| 1-(2,6-Difluorbenzoyl)-3-[4-(2,2-difluorcyclopropyloxy)-3,5-dimethylphenyl]-harnstoff | 0,1 | 100 |
| 1-(2-Chlorbenzoyl)-3-[4-(2,2-difluorcyclopropyloxy)-3,5-dimethylphenyl]-harnstoff | 0,1 | 100 |
| 1-[3,5-Dichlor-4-(2,2-difluorcyclopropyloxy)-phenyl]-3-(2,6-difluorbenzoyl)-harnstoff | 0,1 | 100 |
| 1-[3,5-Dichlor-4-(2,2-difluorcyclopropyloxy)-phenyl]-3-(2-chlorbenzoyl)-harnstoff | 0,1 | 100 |
| 1-(2-Chlor-6-fluorbenzoyl)-3-[4-(2,2-difluorcyclopropyloxy)-3,5-dimethyl-phenyl]-harnstoff | 0,1 | 100 |
| 1-(2,6-Dichlorbenzoyl)-3-[4-(2,2-difluorcyclopropyloxy)-3,5-dimethylphenyl]-harnstoff | 0,1 | 100 |
| 1-[4-(2,2-Difluorcyclopropyloxy)-3,5-dimethylphenyl]-3-(2-fluorbenzoyl)-harnstoff | 0,1 | 100 |
| 1-[4-(2,2-Difluorcyclopropyloxy)-3,5-dimethylphenyl]-3-(2-methylbenzoyl)-harnstoff | 0,1 | 100 |
| 1-(2-Brombenzoyl)-3-[4-(2,2-difluorcyclopropyloxy)-3,5-dimethylphenyl]-harnstoff | 0,1 | 100 |
| 1-[3-Chlor-4-(2,2-difluorcyclopropyloxy)-phenyl]-3-(2,6-difluorbenzoyl)-harnstoff | 0,1 | 100 |
| 1-[3-Chlor-4-(2,2-difluorcyclopropyloxy)-phenyl]-3-(2-chlorbenzoyl)-harnstoff | 0,1 | 100 |
| 1-[3-Chlor-4-(2,2-difluorcyclopropyloxy)-phenyl]-3-(2-fluorbenzoyl)-harnstoff | 0,1 | 100 |
| 1-[3-Chlor-4-(2,2-difluorcyclopropyloxy)-phenyl]-3-(2-chlor-6-fluorbenzoyl)-harnstoff | 0,1 | 100 |
| 1-(2,6-Difluorbenzoyl)-3-[4-(2,2-difluorcyclopropyloxy)-3-methylphenyl]-harnstoff | 0,1 | 100 |



Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jurgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Erfindungsgemäße Verbindungen | Wirkstoffkonzentration in % | Wirkung in % |
|---|---|---|
| 1-(2-Chlorbenzoyl)-3-[4-(2,2-difluorcyclopropyloxy)-3-methylphenyl]-harnstoff | 0,1 | 100 |
| 1-(2-Chlor-6-fluorbenzoyl)-3-[4-(2,2-difluorcyclopropyloxy)-3-methylphenyl]-harnstoff | 0,1 | 100 |
| 1-[4-(2,2-Difluorcyclopropyloxy)-3-methylphenyl]-3-(2-fluorbenzoyl)-harnstoff | 0,1 | 100 |
| 1-[3,5-Dibrom-4-(2,2-difluorcyclopropyloxy)-phenyl]-3-(2,6-difluorobenzoyl)-harnstoff | 0,1 | 100 |
| 1-(2,6-Difluorbenzoyl)-3-{4-/2-(2,2-difluorcyclopropyl)-ethoxy7-3,5-dimethylphenyl}-harnstoff | 0,1 | 100 |
| 1-(2-Chlorbenzoyl)-3-{4-/2-(2,2-difluorcyclopropyl)-ethoxy7-3,5-dimethylphenyl}-harnstoff | 0,1 | 100 |
| 1-{3,5-Dichlor-4-/2-(2,2-difluorcyclopropyl)-ethoxy7-phenyl}-3-(2,6-difluorbenzoyl)-harnstoff | 0,1 | 100 |
| 1-{3,5-Dichlor-4-/2-(2,2-difluorcyclopropyl)-ethoxy7-phenyl}-3-(2-chlorbenzoyl)-harnstoff | 0,1 | 100 |
| 1-(2,6-Difluorbenzoyl)-3-[4-(2,2-difluorcyclopropyloxy)-3,5-dimethylphenyl]-thioharnstoff | 0,1 | 100 |
| 1-(2,6-Difluorbenzoyl)-3-[4-(2,2-difluorcyclopropyloxy)-phenyl]-thioharnstoff | 0,1 | 100 |
| 1-[4-(2,2-Difluorcyclopropyloxy)-phenyl]-3-(2-methylbenzoyl)-harnstoff | 0,1 | 100 |
| 1-(2,6-Difluorbenzoyl)-3-{4-[3-(2,2--difluorcyclopropyl)-propoxy]-3,5-dimethylphenyl}-harnstoff | 0,1 | 100 |
| 1-(2-Chlorbenzoyl)-3-{4-[3-(2,2-difluorcyclopropyl)-propoxy]-3,5-dimethylphenyl}-harnstoff | 0,1 | 100 |
| 1-{3,5-Dichlor-4-[3-(2,2-difluorcyclopropyl)-propoxy]-phenyl}-3-(2,6-difluorbenzoyl)-harnstoff | 0,1 | 100 |

- 30 -

Postanschrift: Schering Aktiengesellschaft, Postfach 65 03 11, D-1000 Berlin 65 · Für Besucher Berlin-Wedding, Müllerstraße 170-178 Telegramme Scheringchemie Berlin

Vorstand: Dr Herbert Asmis, Dr Christian Brunn, Dr. Heinz Hannse, Horst Kramp, Dr. Klaus Pohle, Dr Horst Witzel Vorsitzender des Aufsichtsrats Hans-Jürgen Hamann Sitz der Gesellschaft: Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank, Berlin, Konto-Nr 70045224, Bankleitzahl 100 202 00 Deutsche Bank Berlin AG Konto-Nr 2415008 Bankleitzahl 100 700 00 Postscheckamt Berlin West, Konto-Nr 1175-101, Bankleitzahl 100 100 10

| Erfindungsgemäße Verbindungen | Wirkstoffkonzentration in % | Wirkung in % |
|---|---|---|
| 1-{3,5-Dichlor-4-[3-(2,2-difluor-cyclopropyl)-propoxy]-phenyl}-3-(2-chlorbenzoyl)-harnstoff | 0,1 | 100 |
| 1-{4-[3-(2,2-Difluorcyclopropyl)-propoxy]-3,5-dimethylphenyl}-3-(2-fluor-benzoyl)-harnstoff | 0,1 | 100 |
| 1-{3,5-Dichlor-4-[3-(2,2-difluor-cyclopropyl)-propoxy]-phenyl}-3-(2-fluorbenzoyl)-harnstoff | 0,1 | 100 |
| 1-{4-[2-(2,2-Difluorcyclopropyl)-ethoxy]-3,5-dimethylphenyl}-3-(2-fluorbenzoyl)-harnstoff | 0,1 | 100 |
| 1-[3,5-Dichlor - 4 - (2,2- difluorcyclopropyloxy)- phenyl] - 3 - (2-fluorbenzoyl)-harnstoff | 0,1 | 100 |
| 1- {3,5-Dichlor- 4 - [2-(2,2-difluor-cyclopropyl) - ethoxy] - phenyl } - 3 - (2-fluorbenzoyl)- harnstoff | 0,1 | 100 |
| 1-(2-Chlorbenzol)-3-[4-(2,2-difluorcyclopropyl-methoxy)-3,5-dimethylphenyl]-harnstoff | 0,1 | 100 |
| 1-(2,6-Difluorbenzoyl)-3-[4-(2,2-difluorcyclopropylmethoxy)-phenyl]-harnstoff | 0,1 | 100 |
| 1-(2-Chlorbenzoyl)-3-[4-(2,2-difluor-cyclopropylmethoxy)-phenyl]-harnstoff | 0,1 | 100 |
| 1-(2,6-Difluorbenzoyl)-3-[4-(2,2-difluorcyclopropylmethoxy)-3,5-dimethylphenyl]-harnstoff | 0,1 | 100 |
| 1-[3,5-Dichlor-4-(2,2-difluorcyclopropylmethoxy)-phenyl]-3-(2,6-difluor-benzoyl)-harnstoff | 0,1 | 100 |
| 1-[3,5-Dichlor-4-(2,2-difluorcyclopropylmethoxy)-phenyl]-3-(2-chlor-benzoyl)-harnstoff | 0,1 | 100 |
| 1-[3-Chlor-4-(2,2-difluorcyclopropyl-methoxy)-phenyl]-3-(2,6-difluor-benzoyl)-harnstoff | 0,1 | 100 |

| Erfindungsgemäße Verbindungen | Wirkstoffkonzentration in % | Wirkung in % |
|---|---|---|
| 1-[3-Chlor-4-(2,2-difluorcyclopropylmethoxy)-phenyl]-3-(2-chlorbenzoyl)-harnstoff | 0,1 | 100 |
| 1-[3-Chlor-4-(2,2-difluorcyclopropylmethoxy)-phenyl]-3-(2-fluorbenzoyl)-harnstoff | 0,1 | 100 |
| 1-[3-Chlor-4-(2,2-difluorcyclopropylmethoxy)-phenyl]-3-(2-chlor-6-fluorbenzoyl)-harnstoff | 0,1 | 100 |
| 1-(2,6-Difluorbenzoyl)-3-[4-(2,2-difluorcyclopropylmethoxy)-3-methylphenyl]-harnstoff | 0,1 | 100 |
| 1-(2-Chlorbenzoyl)-3-[4-(2,2-difluorcyclopropylmethoxy)-3-methylphenyl]-harnstoff | 0,1 | 100 |
| 1-(2-Chlor-6-fluorbenzoyl)-3-[4-(2,2-difluorcyclopropylmethoxy)-3-methyl-phenyl]-harnstoff | 0,1 | 100 |
| 1-[4-(2,2-Difluorcyclopropylmethoxy)-3-methylphenyl]-3-(2-fluorbenzoyl)-harnstoff | 0,1 | 100 |
| 1-[3,5-Dibrom-4-(2,2-difluorcyclopropylmethoxy)-phenyl]-3-(2,6-difluorbenzoyl)-harnstoff | 0,1 | 100 |
| 1-[3,5-Dibrom-4-(2,2-difluorcyclopropylmethoxy)-phenyl]-3-(2-chlorbenzoyl)-harnstoff | 0,1 | 100 |
| 1-[3,5-Dibrom-4-(2,2-difluorcyclopropylmethoxy)-phenyl]-3-(2,6-dichlorbenzoyl)-harnstoff | 0,1 | 100 |
| 1-[3,5-Dibrom-4-(2,2-difluorcyclopropylmethoxy)-phenyl]-3-(2-brombenzoyl)-harnstoff | 0,1 | 100 |
| 1-(2,6-Difluorbenzoyl)-3-[4-(2,2-difluorcyclopropylmethoxy)-3,5-dijodphenyl]-harnstoff | 0,1 | 100 |
| 1-(2,6-Difluorbenzoyl)-3-[4-(2,2-difluor-1-methylcyclopropylmethoxy)-3,5-dimethylphenyl]-harnstoff | 0,1 | 100 |

- 32 -

| Erfindungsgemäße Verbindungen | Wirkstoffkonzentration in % | Wirkung in % |
|---|---|---|
| 1-(2-Chlorbenzoyl)-3-[4-(2,2-difluor-1-methylcyclopropylmethoxy)-3,5-dimethylphenyl]-harnstoff | 0,1 | 100 |
| 1-[3,5-Dichlor-4-(2,2-difluor-1-methylcyclopropylmethoxy)-phenyl]-3-(2,6-difluorbenzoyl)-harnstoff | 0,1 | 100 |
| 1-[3,5-Dichlor-4-(2,2-difluor-1-methylcyclopropylmethoxy)-phenyl]-3-(2-chlorbenzoyl)-harnstoff | 0,1 | 100 |
| 1-[3,5-Dibrom-4-(2,2-difluorcyclopropylmethoxy)-phenyl]-3-(2-methylbenzoyl)-harnstoff | 0,1 | 100 |
| 1-[3,5-Dichlor-4-(2,2-difluorcyclopropylmethoxy)-phenyl]-3-(2-methylbenzoyl)-harnstoff | 0,1 | 100 |
| 1-[3,5-Dichlor-4-(2,2-difluor-1-methylcyclopropylmethoxy)-phenyl]-3-(2-methylbenzoyl)-harnstoff | 0,1 | 100 |
| 1-(2,6-Difluorbenzoyl)-3-/4-(2,2-difluor-3-methylcyclopropylmethoxy)-3,5-dimethylphenyl7-harnstoff | 0,1 | 100 |
| 1-(2,6-Difluorbenzoyl)-3-/4-(2,2-difluor-3-phenylcyclopropylmethoxy)-3,5-dimethylphenyl7-harnstoff | 0,1 | 100 |
| 1-(2-Chlorbenzoyl)-3-/4-(2,2-difluor-3-phenylcyclopropylmethoxy)-3,5-dimethylphenyl7-harnstoff | 0,1 | 100 |
| 1-(2-Chlorbenzoyl)-3-/4-(2,2-difluor-3-methylcyclopropylmethoxy)-3,5-dimethylphenyl7-harnstoff | 0,1 | 100 |
| 1-/3,5-Dichlor-4-(2,2-difluor-3-methylcyclopropylmethoxy)-phenyl7-3-(2,6-difluorbenzoyl)-harnstoff | 0,1 | 100 |
| 1-/3,5-Dichlor-4-(2,2-difluor-3-phenylcyclopropylmethoxy)-phenyl7-3-(2,6-difluorbenzoyl)-harnstoff | 0,1 | 100 |
| 1-/4-(2,2-Difluorcyclopropylmethoxy)-3,5-dimethylphenyl7-3-(2-fluorbenzoyl)-harnstoff | 0,1 | 100 |
| 1-/3,5-Dichlor-4-(2,2-difluorcyclopropylmethoxy)-phenyl7-3-(2-fluorbenzoyl)-harnstoff | 0,1 | 100 |
| 1-/3,5-Dichlor-4-(2,2-difluor-3-methylcyclopropylmethoxy)-phenyl7-3-(2-chlorbenzoyl)-harnstoff | 0,1 | 100 |
| 1-/3,5-Dichlor-4-(2,2-difluor-3-phenylcyclopropylmethoxy)-phenyl7-3-(2-chlorbenzoyl)-harnstoff | 0,1 | 100 |

- 33 -

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008 Bankleitzahl 100 700 00 Postscheckamt Berlin West, Konto-Nr 1175-101, Bankleitzahl 100 100 10

| Erfindungsgemäße Verbindungen | Wirkstoffkonzentration in % | Wirkung in % |
|---|---|---|
| 1-[3,5-Dibrom-4-(2,2-difluor-1-methylcyclopropylmethoxy)-phenyl]-3-(2-chlorbenzoyl)- harnstoff | 0,1 | 100 |
| 1-[4-(2,2-Difluor-1-methylcyclopropylmethoxy)- 3,5-dimethylphenyl)-3-(2-fluorbenzoyl)- harnstoff | 0,1 | 100 |
| 1-(2,6-Difluorbenzoyl)-3-[4-(2,2-difluorcyclopropylmethoxy)-3,5- difluorphenyl] - harnstoff | 0,1 | 100 |
| 1-[4-(2,2-Difluorcyclopropylmethoxy) -3,5-difluorphenyl] -3-(2-fluorbenzoyl)-harnstoff | 0,1 | 100 |
| 1-(2-Chlorbenzoyl)-3-[4-(2,2-difluorcyclopropylmethoxy)-3,5-difluorphenyl]-harnstoff | 0,1 | 100 |
| 1-[3,5-Dibrom- 4 -(2,2-difluorcyclopropylmethoxy)- phenyl] - 3 -(2-fluorbenzoyl)-harnstoff | 0,1 | 100 |
| 1-[3,5-Dichlor- 4 - (2,2-difluor- 3 -methylcyclopropylmethoxy)- phenyl] - 3 -(2-fluorbenzoyl)- harnstoff | 0,1 | 100 |
| 1-[3,5-Dibrom-4-(2,2-difluor-3-methylcyclopropylmethoxy)- phenyl] -3- (2-fluorbenzoyl)- harnstoff | 0,1 | 100 |
| 1-[3,5-Dibrom-4- (2,2-difluor-1-methylcyclopropylmethoxy)- phenyl] -3-(2-fluorbenzoyl)- harnstoff | 0,1 | 100 |
| 1-[3,5-Dibrom-4-(2,2-difluor-3-methylcyclopropylmethoxy)- phenyl] -3-(2,6-difluorbenzoyl)- harnstoff | 0,1 | 100 |
| 1-[3,5-Dibrom-4-(2,2-difluor-3-methylcyclopropylmethoxy)- phenyl] -3-(2-chlorbenzoyl)- harnstoff | 0,1 | 100 |
| 1-[3,5-Dibrom-4-(2,2-difluor-1-methylcyclopropylmethoxy)-phenyl] -3-(2,6-difluorbenzoyl)- harnstoff | 0,1 | 100 |

- 34 -

| Erfindungsgemäße Verbindungen | Wirkstoffkonzentration in % | Wirkung in % |
|---|---|---|
| 1-/4-(1-Chlor-2,2-difluorcyclopropylmethoxy)-3,5-dimethylphenyl/-3-(2,6-difluorbenzoyl)-harnstoff | 0,01 | 100 |
| 1-/4-(1-Chlor-2,2-difluorcyclopropylmethoxy)-3,5-dimethylphenyl/-3-(2-chlorbenzoyl)-harnstoff | 0,01 | 100 |
| 1-/4-(1-Chlor-2,2-difluorcyclopropylmethoxy)-3,5-dimethylphenyl/-3-(2-fluorbenzoyl)-harnstoff | 0,01 | 100 |
| 1-/4-(1-Chlor-2,2-difluorcyclopropylmethoxy)-3,5-dimethylphenyl/-3-(2-chlor-6-fluorbenzoyl)-harnstoff | 0,01 | 100 |
| 1-(2,6-Difluorbenzoyl)-3-/4-(2,2-difluor-3,3-dimethylcyclopropylmethoxy)-3,5-dimethylphenyl/-harnstoff | 0,01 | 100 |
| 1-(2-Chlorbenzoyl)-3-/4-(2,2-difluor-3,3-dimethylcyclopropylmethoxy)-3,5-dimethylphenyl/-harnstoff | 0,01 | 100 |
| 1-/3,5-Dichlor-4-(2,2-difluor-3,3-dimethylcyclopropylmethoxy)-phenyl/-3-(2,6-difluorbenzoyl)-harnstoff | 0,01 | 100 |
| 1-(2-Chlorbenzoyl)-3-/3,5-dichlor-4-(2,2-difluor-3,3-dimethylcyclopropylmethoxy)-phenyl/-harnstoff | 0,01 | 100 |

- 35 -

Postanschrift: Schering Aktiengesellschaft, Postfach 65 03 11, D-1000 Berlin 65 · Fur Besucher: Berlin-Wedding, Müllerstraße 170-178 Telegramme Scheringchemie Be
Vorstand: Dr. Heribert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Ham
Sitz der Gesellschaft: Berlin und Bergkamen Handelsregister: AG Charlottenburg 93 HRB 283 und AG Kamen HRB 0061 Berliner Commerzbank AG, Berlin Konto 108700600, Bankleitzahl 100 400 00 Berliner Handels- und Frankfurter Bank, Berlin Konto-Nr. 70045224, Bankleitzahl 100 202 00 Deutsche Bank Berlin AG, Konto 2415008 Bankleitzahl 100 700 00 Postscheckamt Berlin West, Konto-Nr. 11 75-101, Bankleitzahl 100 100 10

| Erfindungsgemäße Verbindungen | Wirkstoffkonzentration in % | Wirkung in % |
|---|---|---|
| 1-/4-(1-Chlor-2,2-difluorcyclopropylmethoxy)-3,5-dichlorphenyl7-3-(2,6-difluorbenzoyl)-harnstoff | 0,01 | 100 |
| 1-/4-(1-Chlor-2,2-difluorcyclopropylmethoxy)-3,5-dichlorphenyl7-3-(2-chlorbenzoyl)-harnstoff | 0,01 | 100 |
| 1-/4-(1-Chlor-2,2-difluorcyclopropylmethoxy)-3,5-dichlorphenyl7-3-(2-chlor-6-fluorbenzoyl)-harnstoff | 0,01 | 100 |
| 1-/4-(1-Chlor-2,2-difluorcyclopropylmethoxy)-3,5-dichlorphenyl7-3-(2-fluorbenzoyl)-harnstoff | 0,01 | 100 |
| 1-/4-(1-Chlor-2,2-difluorcyclopropylmethoxy)-3,5-dichlorphenyl7-3-(2,6-difluorbenzoyl)-thioharnstoff | 0,01 | 100 |
| 1-(2,6-Difluorbenzoyl)-3-[4-(2,2-difluorcyclopropyloxy)-3,5-dimethylphenyl]-harnstoff | 0,01 | 100 |
| 1-[3,5-Dichlor-4-(2,2-difluorcyclopropyloxy)-phenyl]-3-(2,6-difluorbenzoyl)-harnstoff | 0,01 | 85 |
| 1-(2,6-Difluorbenzoyl)-3-[4-(2,2-difluorcyclopropylmethoxy)-3,5-dimethylphenyl]-harnstoff | 0,01 | 80 |
| 1-[3,5-Dichlor-4-(2,2-difluorcyclopropylmethoxy)-phenyl]-3-(2,6-difluorbenzoyl)-harnstoff | 0,01 | 100 |
| 1-/3,5-Dichlor-4-(2,2-difluorcyclopropylmethoxy)-phenyl7-3-(2-chlorbenzoyl)-harnstoff | 0,01 | 100 |
| 1-/3,5-Dibrom-4-(2,2-difluorcyclopropylmethoxy)-phenyl7-3-(2,6-difluorbenzoyl)-harnstoff | 0,01 | 100 |

- 36 -

Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jurgen Hamann Sitz der Gesellschaft Berlin und Bergkamen · Handelsregister AG Charlottenburg 93 HRB 283 und AG Kamen HRB 0061 · Berliner Commerzbank AG, Berlin, Konto-Nr 108700600, Bankleitzahl 100 400 00 · Berliner Handels- und Frankfurter Bank, Berlin, Konto-Nr 70045224, Bankleitzahl 100 202 00 · Deutsche Bank Berlin AG, Konto-Nr 2415008, Bankleitzahl 100 700 00 · Postscheckamt Berlin West, Konto-Nr 1175-101 Bankleitzahl 100 100 10

| Erfindungsgemäße Verbindungen | Wirkstoffkonzentration in % | Wirkung in % |
|---|---|---|
| 1-(2,6-Difluorbenzoyl)-3-/4-(2,2-difluor-1-methylcyclopropylmethoxy)-3,5-dimethylphenyl7-harnstoff | 0,01 | 100 |
| 1-/3,5-Dichlor-4-(2,2-difluorcyclopropylmethoxy)-phenyl7-3-(2-fluorbenzoyl)-harnstoff | 0,01 | 100 |
| 1-/3,5-Dichlor-4-(2,2-difluor-3-methylcyclopropylmethoxy)-phenyl7-3-2,6-difluorbenzoyl)-harnstoff | 0,01 | 100 |
| 1-(3,5-Dichlor-4-cyclopropylmethoxy-phenyl)-3-(2,6-difluorbenzoyl)-harnstoff | 0,01 | 100 |
| 1-(3,5-Dichlor-4-cyclopropylmethoxy-phenyl)-3-(2-fluorbenzoyl)-harnstoff | 0,01 | 100 |
| **VERGLEICHSMITTEL** | | |
| N-(2,6-Difluorbenzoyl)-N'-(p-chlorphenyl)-harnstoff (gemäß DE-OS 21 23 236) | 0,01 | 0 |
| N-(2-Chlorbenzoyl)-N'-(p-trifluormethoxyphenyl)-harnstoff (gemäß DE-OS 26 01 780) | 0,01 | 50 |



Postanschrift: Schering Aktiengesellschaft, Postfach 65 03 11, D-1000 Berlin 65 · Fur Besucher: Berlin-Wedding Mullerstraße 170-178 Telefon: (030) 468-0
Telegramme Scheringchemie Berlin Telex 18203-0 sch d
Vorstand Herbert Asmis Christian Bruhn Heinz Hannse Horst Kramp Klaus Pohle Horst Witzel Vorsitzender des Aufsichtsrats Klaus Subietzki Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00

BEISPIEL 105

Abtötende Wirkung auf Larven (L3) des Mexikanischen Bohnenkäfers (Epilachna varivestis)

Die erfindungsgemäßen Substanzen wurden als wäßrige Emulsionen mit den Wirkstoffkonzentrationen 0,01% und 0,001% eingesetzt. Ebenso wurden die Vergleichsmittel als wäßrige Suspensionen eingesetzt.

In diese Wirkstoffzubereitungen wurden Buschbohnenpflanzen (Phaseolus vulgaris) im Primärblattstadium getaucht. Pro Versuchsglied wurden drei Pflanzenstengel mit insgesamt 6 Primärblättern in mit Wasser gefüllte Glasvasen eingestellt und in Glaszylindern eingekäfigt. Nach dem Antrocknen der Präparatebrühe auf den behandelten Blättern wurden je 5 Larven des Mexikanischen Bohnenkäfers (Epilachna varivestis) im 3. Larvenstadium in die Glaszylinder eingezählt und für 5 Tage darin unter Langtagbedingungen gehalten.

Kriterium für die Wirkungsbeurteilung war die Mortalität der Larven in % nach 5-tägiger Versuchsdauer.

| Erfindungsgemäße Verbindungen | Wirkstoffkonzentration in % | Wirkung in % |
|---|---|---|
| 1-(2,6-Difluorbenzoyl)-3-[4-(2,2-difluorcyclopropyloxy)-3,5-dimethylphenyl]-harnstoff | 0,01<br>0,001 | 100<br>80 |
| 1-(2,6-Difluorbenzoyl)-3-[4-(2,2-difluorcyclopropylmethoxy)-3,5-dimethylphenyl]-harnstoff | 0,01<br>0,001 | 80<br>60 |
| 1-[3,5-Dichlor-4-(2,2-difluorcyclopropylmethoxy)-phenyl]-3-(2,6-difluorbenzoyl)-harnstoff | 0,01<br>0,001 | 100<br>100 |
| 1-/3,5-Dichlor-4-(2,2-difluorcyclopropylmethoxy)-phenyl7-3-(2-chlorbenzoyl)-harnstoff | 0.01<br>0,001 | 100<br>100 |
| 1-/3,5-Dibrom-4-(2,2-difluorcyclopropylmethoxy)-phenyl7-3-(2,6-difluorbenzoyl)-harnstoff | 0,01<br>0,001 | 100<br>100 |
| 1-(2,6-Difluorbenzoyl)-3-/4-(2,2-difluor-1-methylcyclopropylmethoxy)-3,5-dimethylphenyl7-harnstoff | 0,01<br>0,001 | 100<br>90 |
| 1-/3,5-Dichlor-4-(2,2-difluorcyclopropylmethoxy)-phenyl7-3-(2-fluorbenzoyl)-harnstoff | 0,01<br>0,001 | 100<br>100 |
| 1-/3,5-Dichlor-4-(2,2-difluor-3-methylcyclopropylmethoxy)-phenyl7-3-2,6-difluorbenzoyl)-harnstoff | 0,01<br>0,001 | 100<br>100 |

- 37a -

Postanschrift: Schering Aktiengesellschaft, Postfach 65 03 11, D-1000 Berlin 65 · Fur Besucher Berlin-Wedding Mullerstraße 170-178 · Telefon (030) 4 68-0
Telegramme Scheringchemie Berlin Telex 1 82 03-0 sch d

Vorstand Herbert Asmis Christian Bruhn Heinz Hannse Horst Kramp Klaus Pohle Horst Witzel Vorsitzender des Aufsichtsrats Klaus Subjetzki Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postgiroamt Berlin West Konto-Nr 1175-101 Bankleitzahl 100 100 10

VERGLEICHSMITTEL

| | | |
|---|---|---|
| N-(2,6-Difluorbenzoyl)-N'-(p-chlorphenyl)-harnstoff (gemäß DE-OS 21 23 236) | 0,01 | 100 |
| | 0,001 | 0 |
| N-(2-Chlorbenzoyl)-N'-(p-trifluormethoxy-phenyl)-harnstoff (gemäß DE-OS 26 01 780) | 0,01 | 40 |
| | 0,001 | 0 |



Postanschrift: Schering Aktiengesellschaft, Postfach 65 03 11, D-1000 Berlin 65

BEISPIEL 106

Ovizide Wirkung auf Eigelege des Mexikanischen Bohnenkäfers (Epilachna varivestis)

Die erfindungsgemäßen Verbindungen wurden als wäßrige Emulsionen mit der Wirkstoffkonzentration 0,01 % eingesetzt. Ebenso wurden die Vergleichsmittel als wäßrige Suspensionen eingesetzt.

In diese Wirkstoffzubereitungen wurden 1 Tag alte Eigelege, die von befruchteten Käferweibchen auf Buschbohnenblätter abgesetzt worden waren, bis zur völligen Benetzung getaucht und für 7 Tage in geschlossenen Petrischalen deponiert.

Kriterium für die Wirkungsbeurteilung war die Prozentuale Schlupfverhinderung im Vergleich zu unbehandelten Eigelegen.

| Erfindungsgemäße Verbindungen | Wirkstoffkonzentration in % | Wirkung in % |
|---|---|---|
| 1-(2,6-Difluorbenzoyl)-3-[4-(2,2-difluorcyclopropyloxy)-3,5-dimethylphenyl]-harnstoff | 0,01 | 95 |
| 1-(2,6-Difluorbenzoyl)-3-[4-(2,2-difluorcyclopropylmethoxy)-3,5-dimethylphenyl]-harnstoff | 0,01 | 100 |
| 1-[3,5-Dichlor-4-(2,2-difluorcyclopropylmethoxy)-phenyl]-3-(2,6-difluorbenzoyl)-harnstoff | 0,01 | 100 |
| 1-[3,5-Dichlor-4-(2,2-difluorcyclopropylmethoxy)-phenyl]-3-(2-chlorbenzoyl)-harnstoff | 0,01 | 100 |
| 1-[3,5-Dibrom-4-(2,2-difluorcyclopropylmethoxy)-phenyl]-3-(2,6-difluorbenzoyl)-harnstoff | 0,01 | 80 |
| 1-(2,6-Difluorbenzoyl)-3-[4-(2,2-difluor-1-methylcyclopropylmethoxy)-3,5-dimethylphenyl]-harnstoff | 0,01 | 100 |
| 1-[3,5-Dichlor-4-(2,2-difluor-3-methylcyclopropylmethoxy)-phenyl]-3-2,6-difluorbenzoyl)-harnstoff | 0,01 | 100 |



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand: Dr Herbert Asmis, Dr Christian Bruhn, Dr Heinz Hannse, Horst Kramp, Dr Klaus Pohle, Dr Horst Witzel · Vorsitzender des Aufsichtsrats Hans-Jürgen Hamann
Sitz der Gesellschaft Berlin und Bergkamen Handelsregister: AG Charlottenburg 93 HRB 283 und AG Kamen HRB 0061 · Berliner Commerzbank AG, Berlin Konto-Nr 108700600 Bankleitzahl 100 400 00 Berliner Handels- und Frankfurter Bank, Berlin, Konto-Nr. 70045224, Bankleitzahl 100 202 00 Deutsche Bank Berlin AG Konto-Nr 2415008 Bankleitzahl 100 700 00 Postscheckamt Berlin West Konto-Nr. 11 75-101, Bankleitzahl 100 100 10

| VERGLEICHSMITTEL | Wirkstoffkonzentration in % | Wirkung in % |
|---|---|---|
| N-(2,6-Difluorbenzoyl)-N'-(p-chlorphenyl)-harnstoff (gemäß DE-OS 21 23 236) | 0,01 | 50 |
| N-(2-Chlorbenzoyl)-N'-(p-trifluormethoxyphenyl)-harnstoff (gemäß DE-OS 26 01 780) | 0,01 | 0 |
| alle folgenden gemäß DE-OS 29 28 410: | | |
| 1-(4-(2,2-Dichlorcyclopropylmethoxy)-3,5-dimethyl-phenyl)-3-(2,6-dichlorbenzoyl)-harnstoff | 0.01 | 0 |
| 1-(3,5-Dichlor-4-(2,2-dichlorcyclopropylmethoxy)-phenyl)-3-(2,6-dichlorbenzoyl)-harnstoff | 0.01 | 0 |
| 1-(3-Chlor-4-(2,2-dichlorcyclopropylmethoxy)-phenyl)-3-(2-chlorbenzoyl)-harnstoff | 0.01 | 0 |
| 1-(3-Chlor-4-(2,2-dichlorcyclopropylmethoxy)-phenyl)-3-(2-chlor-6-fluorbenzoyl)-harnstoff | 0.01 | 0 |
| 1-(3,5-Dichlor-4-(2,2-dichlorcyclopropylmethoxy)-phenyl)-3-(2-methylbenzoyl)-harnstoff | 0.01 | 0 |
| 1-(3-Chlor-4-(2,2-dichlorcyclopropylmethoxy)-phenyl)-3-(2-methylbenzoyl)-harnstoff | 0.01 | 0 |
| 1-(2-Chlorbenzoyl)-3-(3,5-dichlor-4-(2,2-dichlor-cyclopropylmethoxy)-phenyl)-harnstoff | 0.01 | 0 |
| 1-(2-Chlor-6-fluorbenzoyl)-3-(3,5-dichlor-4-(2,2-dichlorcyclopropylmethoxy)-phenyl)-harnstoff | 0.01 | 0 |
| 1-(3,5-Dichlor-4-(2,2-dichlorcyclopropylmethoxy)-phenyl)-3-(2,6-difluorbenzoyl)-harnstoff | 0.01 | 0 |

- 40 -

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin

Vorstand: Dr. Heribert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

## BEISPIEL 107

Ovizide Wirkung auf Eigelege der Ägyptischen Baumwoll-Eule (Spodoptera littoralis)

Die erfindungsgemäßen Verbindungen wurden als wäßrige Emulsionen mit der Wirkstoffkonzentration 0,0016 % eingesetzt. Ebenso wurden die Vergleichsmittel als wäßrige Suspensionen eingesetzt. In diese Wirkstoffzubereitung wurden 1 Tage alte Eigelege, die von befruchteten Falterweibchen auf Filterpapier abgesetzt worden waren, bis zur völligen Benetzung getaucht und für 4 Tage in geschlossenen Petrischalen deponiert.
Kriterium für die Wirkungsbeurteilung war die prozentuale Schlupfverhinderung im Vergleich zu unbehandleten Eigelegen.

| Erfindungsgemäße Verbindungen | Wirkstoffkonzentration in % | Wirkung in % |
|---|---|---|
| 1-(2,6-Difluorbenzoyl)-3-(4-(2,2-difluorcyclopropylmethoxy)-3,5-dimethylphenyl)-harnstoff | 0,0016 | 80 |
| 1-(3,5-Dichlor-4-(2,2-difluorcyclopropylmethoxy)-phenyl)-3-(2,6-difluorbenzoyl)-harnstoff | 0,0016 | 75 |
| 1/4-(2,2,-Difluorcyclopropylmethoxy)-3,5-dimethyl-phenyl7-3-(2-fluorbenzoyl)-harnstoff | 0,0016 | 80 |
| 1-/3,5-Dichlor-4-(2,2-difluorcyclopropylmethoxy)-phenyl7-3-(2-fluorbenzoyl)-harnstoff | 0,0016 | 100 |
| **Vergleichsmittel** | | |
| N-(2,6-Difluorbenzoyl)-N'-(p-chlorphenyl)-harnstoff (gemäß DE-OS 21 23 236) | 0.0016 | 70 |
| N-(2-Chlorbenzoyl)-N'-(p-trifluormethoxyphenyl)-harnstoff (gemäß DE-OS 25 01 780) | 0.0016 | 70 |
| alle folgenden gemäß DE-OS 29 28 410: | | |
| 1-(4-(2,2-Dichlorcyclopropylmethoxy)-3,5-dimethyl-phenyl)-3-(2,6-dichlorbenzoyl)-harnstoff | 0.0016 | 0 |
| 1-(3,5-Dichlor-4-(2,2-dichlorcyclopropylmethoxy)-phenyl)-3-(2,6-dichlorbenzoyl)-harnstoff | 0.0016 | 0 |
| 1-(2-Chlorbenzoyl)-3-(3,5-dichlor-4-(2,2-dichlor-cyclopropylmethoxy)-phenyl)-harnstoff | 0.0016 | 0 |
| 1-(2-Chlor-6-fluorbenzoyl)-3-(3,5-dichlor-4-(2,2-dichlorcyclopropylmethoxy)-phenyl)-harnstoff | 0.0016 | 0 |
| 1-(3,5-Dichlor-4-(2,2-dichlorcyclopropylmethoxy)-phenyl)-3-(2,6-difluorbenzoyl)-harnstoff | 0.0016 | 0 |



Postanschrift: Schering Aktiengesellschaft, Postfach 65 03 11, D-1000 Berlin 65 · Für Besucher: Berlin-Wedding, Müllerstraße 170-178 Telegramme Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG Berlin Konto-Nr. 108750600 Bankleitzahl 100 400 00 Berliner Handels- und Frankfurter Bank, Berlin Konto-Nr. 70045224 Bankleitzahl 100 202 00 Deutsche Bank Berlin AG Konto-Nr. 2415008 Bankleitzahl 100 700 00 Postscheckamt Berlin West Konto-Nr. 1175-101, Bankleitzahl 100 100 10

| Vergleichsmittel gemäß DE-OS 29 28 410 | Wirkstoffkonzentration in % | Wirkung in % |
|---|---|---|
| 1-(3,5-Dichlor-4-(2,2-dichlorcyclopropylmethoxy)-phenyl)-3-(2-methylbenzoyl)-harnstoff | 0.0016 | 0 |
| 1-(3-Chlor-4-(2,2-dichlorcyclopropylmethoxy)-phenyl)-3-(2-methylbenzoyl)-harnstoff | 0.0016 | 0 |
| 1-(3-Chlor-4-(2,2-dichlorcyclopropylmethoxy)-phenyl)-3-(2-chlorbenzoyl)-harnstoff | 0.0016 | 0 |
| 1-(3-Chlor-4-(2,2-dichlorcyclopropylmethoxy)-phenyl)-3-(2-chlor-6-fluorbenzoyl)-harnstoff | 0.0016 | 0 |



Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin
Vorstand Dr Herbert Asmis Dr Christian Brunn, Dr Heinz Hannse Horst Kramp Dr Klaus Pohle Dr Horst Witzel Vorsitzender des Aufsichtsrats Hans-Jürgen Hamann
Sitz der Gesellschaft Berlin und Bergkamen · Handelsregister AG Charlottenburg 93 HRB 283 und AG Kamen HRB 0061 Berliner Commerzbank AG, Berlin, Konto-Nr 108700600 Bankleitzahl 100 400 00 Berliner Handels- und Frankfurter Bank Berlin Konto-Nr 70045224, Bankleitzahl 100 202 00 Deutsche Bank Berlin AG, Konto-Nr 2415008 Bankleitzahl 100 700 00 Postscheckamt Berlin West Konto-Nr 1175-101, Bankleitzahl 100 100 10

Beispiel 108

Abtötende Wirkung auf Larven (L 3) des Mexikanischen Bohnenkäfers (Epilachna varivestis)

Die Substanzen werden als wäßrige Emulsionen mit der Wirkstoffkonzentration 0,1 % eingesetzt. In diese Wirkstoffzubereitungen werden Buschbohnenpflanzen (Phaseolus vulgaris) im Primärblattstadium getaucht. Pro Versuchsglied werden drei Pflanzenstengel mit insgesamt 6 Primärblättern in mit Wasser gefüllte Glasvasen eingestellt und in Glaszylindern eingekäfigt. Nach dem Antrocknen der Präparatebrühen auf den behandelten Blättern werden je 5 Larven des Mexikanischen Bohnenkäfers (Epilachna varivestis) im dritten Larvenstadium in die Glaszylinder eingezählt und für 5 Tage unter Langtagbedingungen darin gehalten. Kriterium für die Wirkungsbeurteilung ist die Mortalität der Larven in % nach 5-tägiger Versuchsdauer.

| Erfindungsgemäße Verbindungen | Wirkstoffkonzentration in % | Wirkung in % |
|---|---|---|
| 1-[4-(1-Chlor-2,2-difluorcyclopropylmethoxy)-3,5-dimethylphenyl]-3-(2,6-difluorbenzoyl)-harnstoff | 0,1 | 100 |
| 1-[4-(1-Chlor-2,2-difluorcyclopropylmethoxy)-3,5-dimethylphenyl]-3-(2-chlorbenzoyl)-harnstoff | 0,1 | 100 |
| 1-[4-(1-Chlor-2,2-difluorcyclopropylmethoxy)-3,5-dimethylphenyl]-3-(2-fluorbenzoyl)-harnstoff | 0,1 | 100 |

- 43 -

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin

Vorstand: Dr. Heribert Asmis, Dr. Christian Bruhn, Dr. Heinz Hannse, Horst Kramp, Dr. Klaus Ponte, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108730600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West Konto-Nr. 1175-101, Bankleitzahl 100 100 10

| Erfindungsgemäße Verbindungen | Wirkstoffkonzentration in % | Wirkung in % |
|---|---|---|
| 1-/4-(1-Chlor-2,2-difluorcyclopropylmethoxy)-3,5-dimethylphenyl/-3-(2-chlor-6-fluorbenzoyl)-harnstoff | 0,1 | 100 |
| 1-(2,6-Difluorbenzoyl)-3-/4-(2,2-difluor-3,3-dimethylcyclopropylmethoxy)-3,5-dimethylphenyl/-harnstoff | 0,1 | 100 |
| 1-(2-Chlorbenzoyl)-3-/4-(2,2-difluor-3,3-dimethylcyclopropylmethoxy)-3,5-dimethylphenyl/-harnstoff | 0,1 | 100 |
| 1-/3,5-Dichlor-4-(2,2-difluor-3,3-dimethylcyclopropylmethoxy)-phenyl/-3-(2,6-difluorbenzoyl)-harnstoff | 0,1 | 100 |
| 1-(2-Chlorbenzoyl)-3-/3,5-dichlor-4-(2,2-difluor-3,3-dimethylcyclopropylmethoxy)-phenyl/-harnstoff | 0,1 | 100 |
| 1-/4-(1-Chlor-2,2-difluorcyclopropylmethoxy)-3,5-dichlorphenyl/-3-(2,6-difluorbenzoyl)-harnstoff | 0,1 | 100 |
| 1-/4-(1-Chlor-2,2-difluorcyclopropylmethoxy)-3,5-dichlorphenyl/-3-(2-chlorbenzoyl)-harnstoff | 0,1 | 100 |
| 1-/4-(1-Chlor-2,2-difluorcyclopropylmethoxy)-3,5-dichlorphenyl/-3-(2-chlor-6-fluorbenzoyl)-harnstoff | 0,1 | 100 |

- 44

| Erfindungsgemäße Verbindungen | Wirkstoffkonzentration in % | Wirkung in % |
|---|---|---|
| 1-/4-(1-Chlor-2,2-difluorcyclopropyl-methoxy)-3,5-dichlorphenyl/-3-(2-fluor-benzoyl)-harnstoff | 0,1 | 100 |
| 1-/4-(1-Chlor-2,2-difluorcyclopropyl-methoxy)-3,5-dichlorphenyl/-3-(2,6-difluorbenzoyl)-thioharnstoff | 0,1 | 100 |



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin

Vorstand Dr. Herbert Asmis Dr. Christian Bruhn, Dr. Heinz Hannse, Horst Kramp, Dr. Klaus Pohle, Dr. Horst Witzel Vorsitzender des Aufsichtsrats Hans-Jürgen Hamann
Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank, Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175-101 Bankleitzahl 100 100 10

PATENTANSPRÜCHE (für die Vertragsstaaten: BE, CH, DE, FR, IT, LU, NL, SE)

1. Acylharnstoffe der allgemeinen Formel

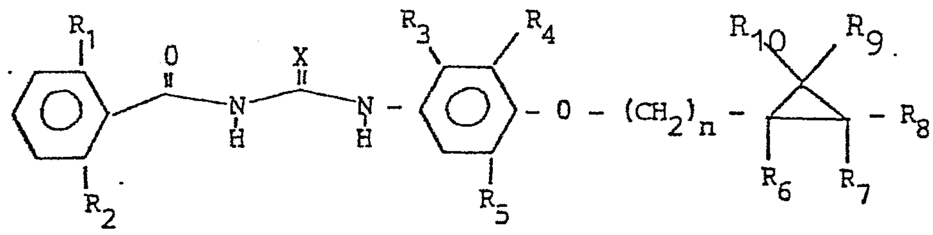

I

in der

$R_1$ Halogen, $C_1$-$C_6$Alkyl oder $C_1$-$C_6$-Halogenalkyl,
$R_2$ Wasserstoff oder Halogen,
$R_3$, $R_4$ und $R_5$ jeweils Wasserstoff, Halogen oder $C_1$-$C_6$-Alkyl,
$R_6$, $R_7$ und $R_8$ jeweils Wasserstoff, $C_1$-$C_6$-Alkyl, Aryl oder Halogen,
$R_9$, $R_{10}$ gleich oder verschieden sind und Wasserstoff oder Fluor,
X Sauerstoff oder Schwefel und
n 0, 1, 2 oder 3
bedeuten.

2. Acylharnstoffe gemäß Anspruch 1, worin
$R_1$ Chlor oder Fluor,
$R_2$ Wasserstoff, Chlor oder Fluor,
$R_3$ Wasserstoff, Chlor oder Methyl,
$R_4$ Wasserstoff, Chlor oder Methyl,
$R_5$ Wasserstoff, Chlor oder Methyl,
$R_6$ Wasserstoff, Chlor oder Methyl,
$R_7$ Wasserstoff oder Methyl,
$R_8$ Wasserstoff oder Methyl,
$R_9$ und $R_{10}$ Fluor,
X Sauerstoff und
n 0, 1, 2 oder 3 bedeuten.



Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 Berliner Commerzbank AG, Berlin, Konto-Nr. 108700600, Bankleitzahl 100 400 00 Berliner Handels- und Frankfurter Bank, Berlin, Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

3. Verfahren zur Herstellung von Acylharnstoffen gemäß Ansprüchen 1 und 2 dadurch gekennzeichnet, daß man

a) Alkoxyaniline der allgemeinen Formel

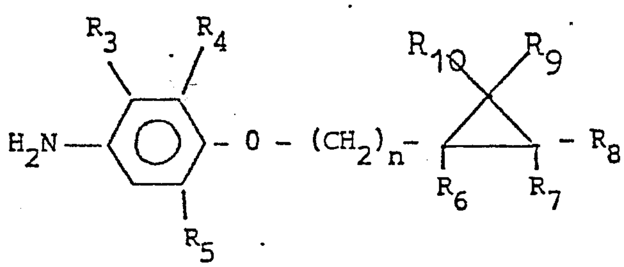 II

mit Benzoylisocyanaten oder Benzoylisothiocyanaten der allgemeinen Formel

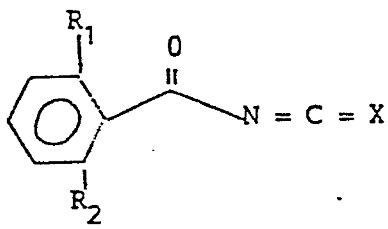 III

gegebenenfalls unter Verwendung eines Lösungsmittels sowie eines Katalysators zur Reaktion bringt oder

b) Alkoxyphenylisocyanate oder Alkoxyphenylisothiocyanate der allgemeinen Formel

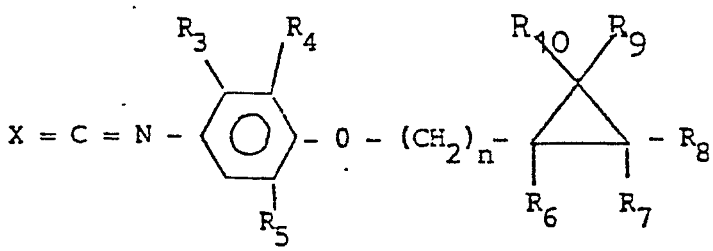 IV

mit Benzamiden der allgemeinen Formel

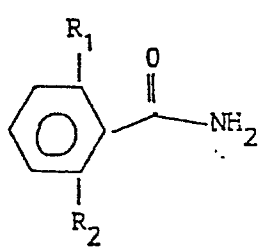 V



**Postanschrift: Schering Aktiengesellschaft, Postfach 65 03 11, D-1000 Berlin 65** · Für Besucher: Berlin-Wedding, Müllerstraße 170-178 Telefon (030) 468-1
Telegramme Scheringchemie Berlin Telex 182 03-0 sch d

Vorstand: Herbert Asmis, Christian Bruhn, Heinz Hannse, Horst Krämer, Klaus Pohle, Horst Witzel · Vorsitzender des Aufsichtsrats: Klaus Subjetzki · Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108700600, Bankleitzahl 100 400 00 · Berliner Handels- und Frankfurter Bank Berlin, Konto-Nr. 70045224, Bankleitzahl 100 202 00 · Deutsche Bank Berlin AG, Konto-Nr. 2415008, Bankleitzahl 100 700 00 · Postgiroamt Berlin West, Konto-Nr. 1175-101, Bankleitzahl 100 100 10

gegebenenfalls in Gegenwart eines Lösungsmittels sowie eines Katalysators umsetzt, wobei $R_1$, $R_2$, $R_3$, $R_4$, $R_5$, $R_6$, $R_7$, $R_8$, $R_9$, $R_{10}$, X und n die oben angegebene Bedeutung haben.

4. Insektizide Mittel, gekennzeichnet durch einen Gehalt an mindestens einer Verbindung gemäß Ansprüchen 1 und 2.

5. Insektizide Mittel gemäß Anspruch 4 in Mischung mit Träger und/oder Hilfsstoffen.

6. Verwendung von Verbindungen gemäß Ansprüchen 1 und 2 zur Bekämpfung von Insekten.



Postanschrift: Schering Aktiengesellschaft, Postfach 65 03 11, D-1000 Berlin 65 · Fur Besucher Berlin-Wedding, Mullerstraße 170-178 Telefon (030) 468-0
Telegramme Scheringchemie Berlin Telex 182 03-0 sch d
Vorstand: Heroert Asmis Christian Bruhn Heinz Hannse Horst Kramp Klaus Pohle Horst Witzel Vorsitzender des Aufsichtsrats Klaus Subjetzki Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postgiroamt Berlin West Konto-Nr 1175-101 Bankleitzahl 100 100 10

PATENTANSPRÜCHE (für den Vertragsstaat AT)

1. Insektizide Mittel, gekennzeichnet durch einen Gehalt an mindestens einem Acvlharnstoff der allgemeinen Formel

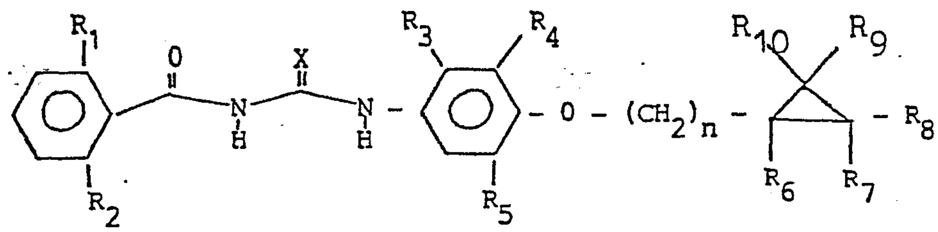

I

in der

$R_1$ Halogen, $C_1$-$C_6$Alkyl oder $C_1$-$C_6$-Halogenalkyl,

$R_2$ Wasserstoff oder Halogen,

$R_3$, $R_4$ und $R_5$ jeweils Wasserstoff, Halogen oder $C_1$-$C_6$-Alkyl,

$R_6$, $R_7$ und $R_8$ jeweils Wasserstoff, $C_1$-$C_6$-Alkyl, Aryl oder Halogen,

$R_9$, $R_{10}$ gleich oder verschieden sind und Wasserstoff oder Fluor,

X Sauerstoff oder Schwefel und

n 0, 1, 2 oder 3

bedeuten, als wirksamen Bestandteil.

2. Mittel gemäß Anspruch 1, gekennzeichnet durch einen Gehalt an mindestens einem Acylharnstoff der im Anspruch 1 angegebenen allgemeinen Formel I, worin

$R_1$ Chlor oder Fluor,

$R_2$ Wasserstoff, Chlor oder Fluor,

$R_3$ Wasserstoff, Chlor oder Methyl,

$R_4$ Wasserstoff, Chlor oder Methyl,

$R_5$ Wasserstoff, Chlor oder Methyl,

$R_6$ Wasserstoff, Chlor oder Methyl,

$R_7$ Wasserstoff oder Methyl,

$R_8$ Wasserstoff oder Methyl,

$R_9$ und $R_{10}$ Fluor,

X Sauerstoff und

n 0, 1, 2 oder 3 bedeuten, als wirksamen Bestandteil.



Postanschrift: Schering Aktiengesellschaft, Postfach 65 03 11, D-1000 Berlin 65 · Fur Besucher: Berlin-Wedding, Müllerstraße 170-178 Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108700600 Bankleitzahl 100 400 00 · Berliner Handels- und Frankfurter Bank, Berlin, Konto-Nr. 70045224 Bankleitzahl 100 202 00 · Deutsche Bank Berlin AG, Konto-Nr. 2415008 Bankleitzahl 100 700 00 · Postscheckamt Berlin West, Konto-Nr. 1175-101 Bankleitzahl 100 100 10

3. Verfahren zur Herstellung von Acylharnstoffen gemäß Ansprüchen 1 und 2 dadurch gekennzeichnet, daß man

a) Alkoxyaniline der allgemeinen Formel

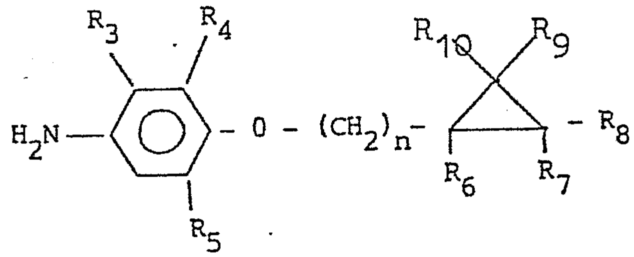

II

mit Benzoylisocyanaten oder Benzoylisothiocyanaten der allgemeinen Formel

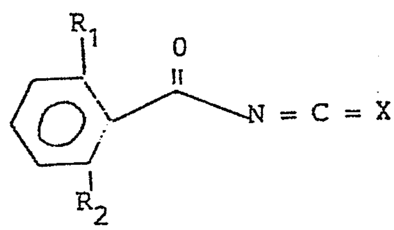

III

gegebenenfalls unter Verwendung eines Lösungsmittels sowie eines Katalysators zur Reaktion bringt oder

b) Alkoxyphenylisocyanate oder Alkoxyphenylisothiocyanate der allgemeinen Formel

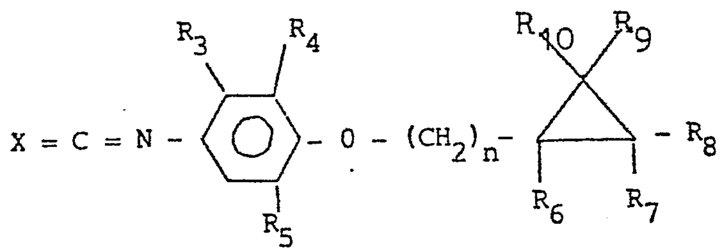

IV

mit Benzamiden der allgemeinen Formel

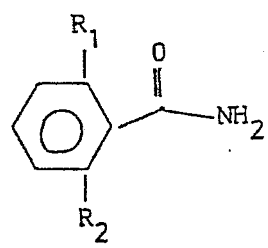

V

- 5

gegebenenfalls in Gegenwart eines Lösungsmittels sowie eines Katalysators umsetzt, wobei $R_1$, $R_2$, $R_3$, $R_4$, $R_5$, $R_6$, $R_7$, $R_8$, $R_9$, $R_{10}$, X und n die oben angegebene Bedeutung haben.

4. Verwendung von Verbindungen gemäß Ansprüchen 1 und 2 zur Bekämpfung von Insekten.



**Postanschrift: Schering Aktiengesellschaft, Postfach 65 03 11, D-1000 Berlin 65** · Für Besucher: Berlin-Wedding Müllerstraße 170-178 Telefon (0 30) 4 68-0
Telegramme Scheringchemie Berlin · Telex 1 82 03-0 sch d

Vorstand Herbert Asmis Christian Bruhn Heinz Hannse Horst Kramp Klaus Pohle, Horst Witzel · Vorsitzender des Aufsichtsrats Klaus Subjetzki · Sitz der Gesellschaft Berlin und Bergkamen · Handelsregister AG Charlottenburg 93 HRB 283 und AG Kamen HRB 0061 · Berliner Commerzbank AG Berlin Konto-Nr 108 [illegible] Bankleitzahl 100 400 00 · Berliner Handels- und Frankfurter Bank Berlin Konto-Nr 70045224 Bankleitzahl 100 202 00 · Deutsche Bank Berlin AG Konto-Nr 24 [illegible] Bankleitzahl 100 700 00 · Postgiroamt Berlin West Konto-Nr 11 75 10 Bankleitzahl 100 100 10